# EUROPEAN PATENT SPECIFICATION

(11) **EP 0 865 492 B1**
(45) Date of publication and mention of the grant of the patent: **11.04.2001**
(21) Application number: 96941758.3
(22) Date of filing: 09.12.1996
(51) Int. Cl.: C12N 15/13, C07K 16/30, A61K 39/395, A61K 51/10, C12Q 1/02, G01N 33/58, G01N 33/68

(54) **SPECIFIC BINDING MEMBERS FOR HUMAN CARCINOEMBRYONIC ANTIGEN, MATERIALS AND METHODS**
SPEZIFISCHE BINDUNGSPARTNER FUER MENSCHLICHES KARZINOEMBRYONISCHES ANTIGEN, SUBSTANZEN UND METHODEN
ELEMENTS DE LIAISON SPECIFIQUES DE L'ANTIGENE CARCINOEMBRYONNAIRE HUMAIN, MATERIAUX ET METHODES

(30) Priority: 07.12.1995 GB 9525004; 23.05.1996 GB 9610824; 11.10.1996 GB 9621295
(43) Date of publication of application: 23.09.1998
(73) Proprietor: Cambridge Antibody Technology Limited, Royston, Cambridgeshire SG8 6JJ (GB)
(72) Inventor: OSBOURN, Jane, Katharine, Cambridge CB4 3DJ (GB); ALLEN, Deborah, Julie, Cambridge CB1 3AL (GB); McCAFFERTY, John, Gerald, Babraham CB2 4AP (GB)
(74) Representative: Walton, Seán Malcolm
(86) International application number: GB9603043
(87) International publication number: WO9720932

(56) References cited:
- WO-A-91/01990
- WO-A-93/11236
- WO-A-95/06067
- WO-A-95/15341
- CANCER RESEARCH, vol. 52, 15 April 1992, pages 2329-2339, XP002027738 M. NAP ET AL.: "Specificity and affinity of monoclonal antibodies against Carcinoembryonic antigen."
- DEVELOPMENTS IN BIOLOGICAL STANDARDIZATION, vol. 66, 1987, pages 429-437, XP000645830 N. EPSTEIN ET AL.: "Production of Carcinoembryonic antigen from a human colon adenocarcinoma cell line. II. Use of monoclonal antibodies to carcinoembryonic antigen purification and characterization."
- THE EMBO JOURNAL, vol. 12, no. 2, 1993, pages 725-734, XP000645476 A.D. GRIFFITHS ET AL.: "Human anti-self antibodies with high specificity from phage display librabries" cited in the application
- THE EMBO JOURNAL, vol. 13, 1994, pages 3245-3260, XP000455240 A.D. GRIFFITHS ET AL.: "Isolation of high affinity human antibodies directly from large synthetic repertoires." cited in the application
- CANCER RESEARCH, vol. 56, no. 13, 1 July 1996, pages 3055-3061, XP000645454 SHI-ZHEN HU ET AL.: "Minibody: A novel engineered anti-carcinoembryonic antigen antibody fragment (single-chaim Fv-CH3) which exhibits rapid, high-level targeting of xenografts."
- IMMUNOTECHNOLOGY (AMSTERDAM) 2 (3). 1996. 181-196. ISSN: 1380-2933, XP000645453 OSBOURN J K ET AL: "Generation of a panel of related human scFv antibodies with high affinities for human CEA."
- BIOCHEM BIOPHYS RES COMMUN 144 (2). 1987. 634-642. CODEN: BBRCA9 ISSN: 0006-291X, XP000645416 OIKAWA S ET AL: "THE CARCINOEMBRYONIC ANTIGEN CEA CONTAINS MULTIPLE IMMUNOGLOBULIN-LIKE DOMAINS."
- HYBRIDOMA, vol. 5 , no. 3, 1986, pages 199-213, XP000647573 R. WELLERSON ET AL.: "Enhanced binding activity observed between anti-carcinoembryonic monoclonal antibodies."

## Description

This invention relates to specific binding members for human carcinoembryonic antigen (CEA) and materials and methods relating thereto.

CEA is a tumour-associated glycoprotein, the expression of which is increased in a number of human carcinomas. CEA is a widely used clinical tumour marker, and antibodies raised against it have been used for imaging (Goldenberg, D.M. Int. J. of Biol. Markers 1992, 7; 183-188) and therapy (e.g. Ledermann et al., Int. J. Cancer 1991, 47; 659-664). CEA is a member of the immunoglobulin superfamily and has homology with a number of other antigens, such as normal cross-reacting antigen (NCA), found on normal tissues (Buchegger, F. et al., 1984, Int. J. Cancer 33; 643-649.).

A number of mouse anti-CEA antibodies exist binding to a range of epitopes on CEA (Hammarstrom et al., 1989, Cancer Res. 49, 4852-4858) and human anti-CEA antibodies have been isolated from human phage display libraries (A.D. Griffiths et al. EMBO J. 12, 1993; 725-734; A.D. Griffiths et al. EMBO J. 13 3245-3260, 1994; WO93/11236) The present invention results from the inventors having obtained the first example of human anti-CEA antibodies with a dissociation constant of less than 10nM for CEA (1 x 10⁻⁸ M) and the first such human anti-CEA antibodies which do not cross-react with cell types which express NCA or with a normal human liver cell line.

Herein it is shown that large universal phage display libraries may be used as a source of human antibodies specific for human CEA. Human antibodies to human CEA with improved properties can then be engineered in a number of ways. In Example 1 it is demonstrated how the affinity of the human anti-CEA antibody can be improved by oligonucleotide directed mutagenesis of the complementarity determining regions (CDR's) of the VH and VL domains of the antibodies. The use of antibody chain shuffling is also demonstrated, for instance combining the VH domains of antibodies derived from one library with the VL domains of another library, thus expanding the pool of VL partners tested for each VH domain. Example 1 also demonstrates the use of this procedure, or a combination of oligonucleotide mutagenesis and VL chain shuffling, to generate new antibodies which have an altered specificity on a range of normal tissues compared to the parental antibody. The antibodies also have an improved affinity for human CEA compared with the parental antibody. It is demonstrated that this procedure is capable of changing the specificity of the original antibody in such a way as to improve its potential performance as a specific tumour targeting agent. Cross-reactivity to a human cell line of normal liver cells is greatly reduced with certain combinations of VH and VL.

The use of anti-CEA antibodies in the treatment and diagnosis of cancer has been the subject of a number of patents (e.g. Matsuoka and Kuroki (1989) Patent no. 4871834; Buchegger and Mach (1991) JP Patent no. 5047507; Chester et al 1995, WO 95/15341). The human antibodies disclosed herein should be valuable for similar applications with the advantage that they will enable the use of repeat treatments due to the absence of the human anti-mouse antibody (HAMA) response (Schroff et al (1985) *Cancer Res* 45: 879-885; DeJager et al (1988) *Proc. Am. Assoc. Cancer Res.* 29:377) . HAMA responses have a range of effects, from neutralisation of the administered antibody leading to a reduced therapeutic dose, through to allergic responses, serum sickness and renal impairment.

It is shown herein that the human antibodies against human CEA can be effective in tumour localisation in a mouse xenograft model of human adenocarcinoma.

WO95/15341 discloses anti-CEA antibody obtained from a phage display library made from mice immunised with human CEA.

Nap *et al., Cancer Research* Vol 55, pp2329-2339, describes analysis of a panel of murine anti-CEA monoclonal antibodies.

WO91/01990 discloses chimeric antibodies against CEA, comprising a mouse variable region and a human constant region.

Epstein *et al.,* Developments in Biological Standardization, 1987, Vol. 66, pp 429-437 describes isolation of murine monoclonal antibodies against CEA from human metastases to the liver of primary colon adenocarcinoma.

### TERMINOLOGY

### Specific binding member

This describes a member of a pair of molecules which have binding specificity for one another. The members of a specific binding pair may be naturally derived or wholly or partially synthetically produced. One member of the pair of molecules has an area on its surface, or a cavity, which specifically binds to and is therefore complementary to a particular spatial and polar organisation of the other member of the pair of molecules. Thus the members of the pair have the property of binding specifically to each other. Examples of types of specific binding pairs are antigen-antibody, biotin-avidin, hormone-hormone receptor, receptor-ligand, enzyme-substrate. This application is concerned with antigen-antibody type reactions.

### Antibody

This describes an immunoglobulin whether natural or partly or wholly synthetically produced. The term also covers any polypeptide or protein having a binding domain which is, or is homologous to, an antibody binding domain. These can be derived from natural sources, or they may be partly or wholly synthetically produced. Examples of antibodies are the immunoglobulin isotypes and their isotypic subclasses; fragments which comprise an antigen binding domain such as Fab, scFv, Fv, dAb, Fd; and diabodies.

It is possible to take monoclonal and other antibodies and use techniques of recombinant DNA technology to produce other antibodies or chimeric molecules which retain the specificity of the original antibody. Such techniques may involve introducing DNA encoding the immunoglobulin variable region, or the complementarity determining regions (CDRs), of an antibody to the constant regions, or constant regions plus framework regions, of a different immunoglobulin. See, for instance, EP-A-184187, GB 2188638A or EP-A-239400. A hybridoma or other cell producing an antibody may be subject to genetic mutation or other changes, which may or may not alter the binding specificity of antibodies produced.

As antibodies can be modified in a number of ways, the term "antibody" should be construed as covering any specific binding member or substance having a binding domain with the required specificity. Thus, this term covers antibody fragments, derivatives, functional equivalents and homologues of antibodies, including any polypeptide comprising an immunoglobulin binding domain, whether natural or wholly or partially synthetic. Chimeric molecules comprising an immunoglobulin binding domain, or equivalent, fused to another polypeptide are therefore included. Cloning and expression of chimeric antibodies are described in EP-A-0120694 and EP-A-0125023.

It has been shown that fragments of a whole antibody can perform the function of binding antigens. Examples of binding fragments are (i) the Fab fragment consisting of VL, VH, CL and CH1 domains; (ii) the Fd fragment consisting of the VH and CH1 domains; (iii) the Fv fragment consisting of the VL and VH domains of a single antibody; (iv) the dAb fragment (Ward, E.S. et al., Nature 341, 544-546 (1989)) which consists of a VH domain; (v) isolated CDR regions; (vi) F(ab')2 fragments, a bivalent fragment comprising two linked Fab fragments (vii) single chain Fv molecules (scFv), wherein a VH domain and a VL domain are linked by a peptide linker which allows the two domains to associate to form an antigen binding site (Bird et al, Science, 242, 423-426, 1988; Huston et al, PNAS USA, 85, 5879-5883, 1988); (viii) bispecific single chain Fv dimers (PCT/US92/09965) and (ix) "diabodies", multivalent or multispecific fragments constructed by gene fusion (WO94/13804; P. Holliger et al Proc. Natl. Acad. Sci. USA 90 6444-6448, 1993).

Diabodies are multimers of polypeptides, each polypeptide comprising a first domain comprising a binding region of an immunoglobulin light chain and a second domain comprising a binding region of an immunoglobulin heavy chain, the two domains being linked (e.g. by a peptide linker) but unable to associate with each other to form an antigen binding site: antigen binding sites are formed by the association of the first domain of one polypeptide within the multimer with the second domain of another polypeptide within the multimer (WO94/13804).

Where bispecific antibodies are to be used, these may be conventional bispecific antibodies, which can be manufactured in a variety of ways (Holliger, P. and Winter G. Current Opinion Biotechnol. 4, 446-449 (1993)), eg prepared chemically or from hybrid hybridomas, or may be any of the bispecific antibody fragments mentioned above. It may be preferable to use scFv dimers or diabodies rather than whole antibodies. Diabodies and scFv can be constructed without an Fc region, using only variable domains, potentially reducing the effects of anti-idiotypic reaction. Other forms of bispecific antibodies include the single chain "Janusins" described in Traunecker et al, Embo Journal; 10, 3655-3659, (1991).

Bispecific diabodies, as opposed to bispecific whole antibodies, may also be particularly useful because they can be readily constructed and expressed in *E.coli.* Diabodies (and many other polypeptides such as antibody fragments) of appropriate binding specificities can be readily selected using phage display (WO94/13804) from libraries. If one arm of the diabody is to be kept constant, for instance, with a specificity directed against antigen X, then a library can be made where the other arm is varied and an antibody of appropriate specificity selected.

### Antigen binding domain

This describes the part of an antibody which comprises the area which specifically binds to and is complementary to part or all of an antigen. Where an antigen is large, an antibody may only bind to a particular part of the antigen, which part is termed an epitope. An antibody binding domain may be provided by one or more antibody variable domains. Preferably, an antigen binding domain comprises an antibody light chain variable region (VL) and an antibody heavy chain variable region (VH).

### Specific

This may be used to refer to the situation in which one member of a specific binding pair will not show any significant binding to molecules other than its specific binding partner(s). The term is also applicable where e.g. an antigen binding domain is specific for a particular epitope which is carried by a number of antigens, in which case the specific binding member carrying the antigen binding domain will be able to bind to the various antigens carrying the epitope.

### Functionally equivalent variant form

This refers to a molecule (the variant) which although having structural differences to another molecule (the parent) retains some significant homology and also at least some of the biological function of the parent molecule, e.g. the ability to bind a particular antigen or epitope. Variants may be in the form of fragments, derivatives or mutants. A variant, derivative or mutant may be obtained by modification of the parent molecule by the addition, deletion, substitution or insertion of one or more amino acids, or by the linkage of another molecule. These changes may be made at the nucleotide or protein level. For example, the encoded polypeptide may be a Fab fragment which is then linked to an Fc tail from another source. Alternatively, a marker such as an enzyme, flourescein, etc, may be linked.

The present invention generally provides a specific binding member (comprising a polypeptide) which comprises a human antibody antigen binding domain which is specific for human CEA.

In one aspect, the binding domain has a dissociation constant for human CEA which is less than 1.0 x 10⁻⁸M, preferably less than 5.0 x 10⁻⁹M.

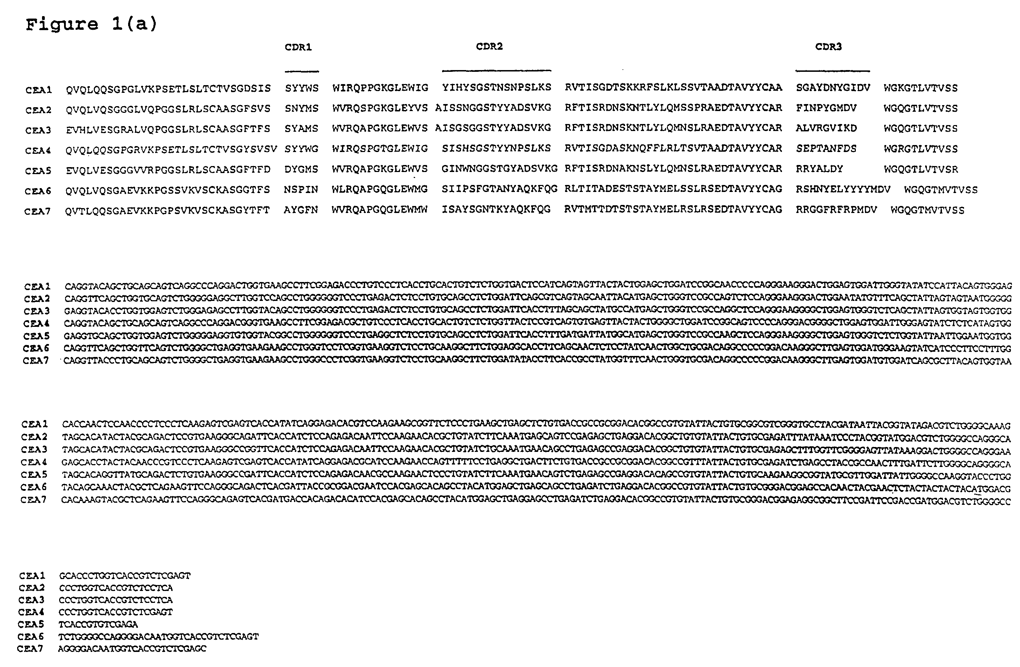

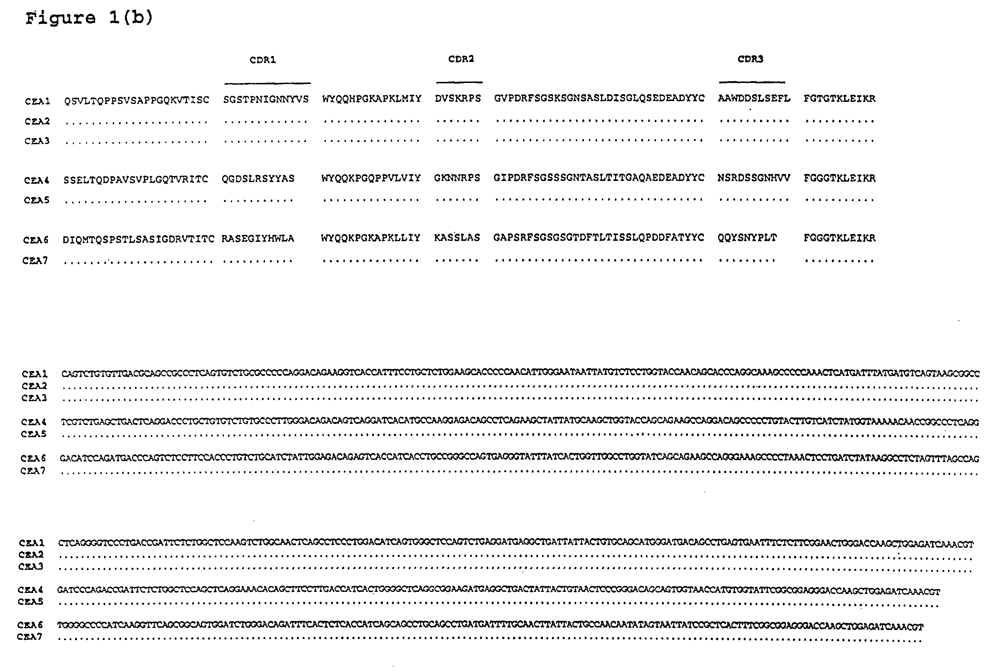

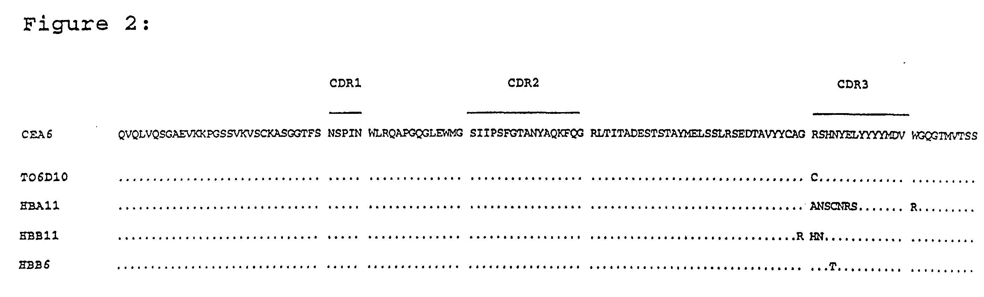

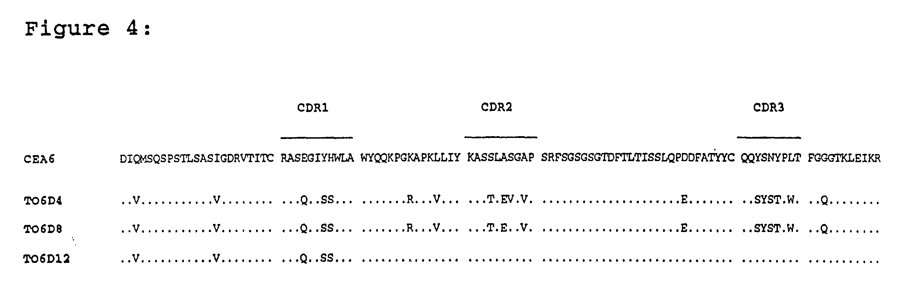

A specific binding member comprising a human antibody antigen binding domain specific for human carcinoembryonic antigen wherein the binding domain has a dissociation constant for human carcinoembryonic antigen which is less than 1.0 x 10⁻⁸M may comprise a binding domain comprising a pairing of VH and VL domains selected from:
i) the VH domain of CEA6, the amino acid sequence of which is shown in Figure 1(a), and the VL domain of CEA6, the amino acid sequence for which is shown in Figure 1(b);
ii) the VH domain of CEA6, the amino acid sequence of which is shown in Figure 1(a), and a VL domain selected from T06D4, T06D8 and T06D12, the amino acid sequences of which are shown in Figure 4;
iii) the VL domain of CEA6, the amino acid sequence of which is shown in Figure 1(b), and a VH domain selected from T06D10, HBA11, HBB11 and HBB6, the amino acid sequences of which are shown in Figure 2; and
iv) that of TO6D11, i.e. the VH domain of T06D10, the amino acid sequence of which is shown in Figure 2, and the VL domain of T06D12, the amino acid sequence of which is shown in Figure 4.

In another aspect, the specific binding member does not or does not significantly bind to or cross-react with human liver cells, for example a human liver cell line. There may be low cross-reactive binding with human liver cells provided it is not significant compared with the binding to human CEA. Thus, the specific binding member may be substantially non-cross-reactive with human liver cells. Likewise, it may not bind or significantly bind other normal tissues or cells such as vascular endothelium, muscle, neutrophils, erythrocytes or lymphocytes. The lack of reactivity with normal lymphocytes and neutrophils is indicative that there is not a high level of crossreactivity with NCA.

A specific binding member comprising a human antibody antigen binding domain specific for human carcinoembryonic antigen, wherein the binding domain is substantially non-cross-reactive with human liver cells may comprise a pairing of VH and VL domains selected from:
i) the VH domain of CEA6, the amino acid sequence of which is shown in Figure 1(a), and a VL domain selected from T06D4 and T06D12, the amino acid sequences of which are shown in Figure 4;
ii) the VL domain of CEA6, the amino acid sequence of which is shown in Figure 1(b), and the VH domain of TO6D10, the amino acid sequence of which is shown in Figure 2; and
iii) that of TO6D11, i.e. the VH domain of T06D10, the amino acid sequence of which is shown in Figure 2, and the VL domain of TO6D12, the amino acid sequence of which is shown in Figure 4.

The specific binding member may bind cell-associated CEA or soluble CEA. It may bind preferentially to cell-associated CEA.

A specific binding members comprising a human antibody antigen binding domain specific for human carcinoembryonic antigen, wherein the binding domain binds to cell-associated human carcinoembryonic antigen preferentially over soluble human carcinoembryonic antigen may comprise a pairing of VH and VL domains selected from:
i) the VH domain of CEA6, the amino acid sequence of which is shown in Figure 1(a), and. the VL domain of CEA6, the amino acid sequence for which is shown in Figure 1(b);
ii) the VH domain of CEA6, the amino acid sequence of which is shown in Figure 1(a), and a VL domain selected from T06D4 and T06D12, the amino acid sequences of which are shown in Figure 4;
iii) the VL domain of CEA6, the amino acid sequence of which is shown in Figure 1(b), and a VH domain selected from TO6D10 and HBB11, the amino acid sequences of which are shown in Figure 2; and
iv) that of TO6D11, i.e. the VH domain of TO6D10, the amino acid sequence of which is shown in Figure 2, and the VL domain of TO6D12, the amino acid sequence of which is shown in Figure 4.

A specific binding member according to a further aspect of the present invention is specific for a carbohydrate epitope of human CEA. Examples include specific binding members comprising the VH and VL pairing of any of CEA1, CEA2, CEA3, CEA4 and CEA5, the amino acid sequences of the VH domains of which are shown in Figure 1(a) and the amino acid sequences of the VL domains of which are shown in Figure 1(b).

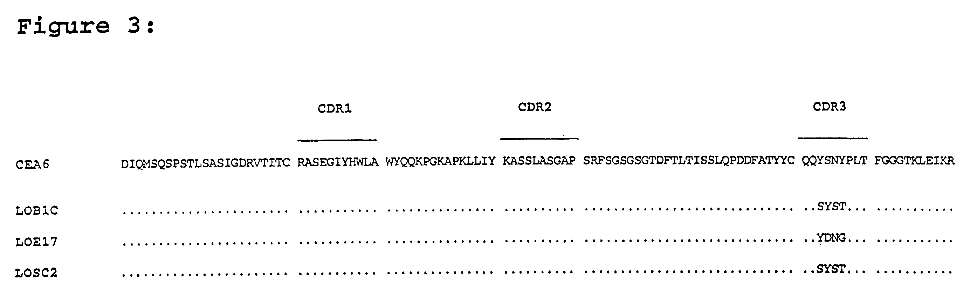

In a further aspect the present invention provides a specific binding member comprising a human antibody antigen binding domain which binds, preferably specific for, the A3-B3 extracellular domain of human CEA. Such a specific binding member may comprise a pairing of VH and VL domains selected from:
i) the VH domain of CEA6, the amino acid sequence of which is shown in Figure 1(a), and the VL domain of CEA6, the amino acid sequence for which is shown in Figure 1(b);
ii) the VH domain of CEA6, the amino acid sequence of which is shown in Figure 1(a), and a VL domain selected from T06D4, TO6D8 and T06D12, the amino acid sequences of which are shown in Figure 4;
iii) the VL domain of CEA6, the amino acid sequence of which is shown in Figure 1(b), and a VH domain selected from HBA11, HBB11 and HBB6, the amino acid sequences of which are shown in Figure 2;
iv) that of TO6D11, i.e. the VH domain of TO6D10, the amino acid sequence of which is shown in Figure 2, and the VL domain of TO6D12, the amino acid sequence of which is shown in Figure 4; and
v) the VH domain of CEA6, the amino acid sequence of which is shown in Figure 1(a), and a VL domain selected from LOB1C, LOE17 and LOSC2, the amino acid sequences of which are shown in Figure 3.

The specific binding member may be in the form of an antibody fragment such as single chain Fv (scFv). Other types of antibody fragments may also be utilised such as Fab, Fab', F(ab')2, Fabc, Facb or a diabody (G. Winter and C. Milstein Nature 349, 293-299,1991; WO94/13804). The specific binding member may be in the form of a whole antibody. The whole antibody may be in any of the forms of the antibody isotypes eg IgG, IgA, IgD, IgE and IgM and any of the forms of the isotype subclasses eg IgGl or IgG4.

The specific binding member may also be in the form of an engineered antibody e.g. a bispecific antibody molecule (or a fragment such as F(ab')2) which has one antigen binding arm (i.e. specific domain) against CEA and another arm against a different specificity, or a bivalent or multivalent molecule.

In addition to antibody sequences, the specific binding member may comprise other amino acids, e.g. forming a peptide or polypeptide, or to impart to the molecule another functional characteristic in addition to ability to bind antigen. For example, the specific binding member may comprise a label, an enzyme or a fragment thereof and so on.

The binding domain may comprise part or all of a VH domain encoded by a germ line segment or a re-arranged gene segment. The binding domain may comprise part or all of a VL kappa domain or a VL lambda domain.

The binding domain may comprise a VH1, VH3 or VH4 gene sequence of one of the following germ lines: the DP71 germ line; the DP47 germ line; the DP67 germ line; the DP32 germ line; the DP10 germ line or the DP14 germ line; or a re-arranged form thereof. The 'DP' nomenclature is described in Tomlinson I.M. et al, (1992) J. Mol. Biol. 227: 776-798.

The binding domain may comprise a Vl1, Vl3 or Vk1 gene sequence of one of the following germ lines: the germ line DPL5; the DPL2 germ line; the germ line DPL16; the germ line L12a; or a re-arranged form thereof.

The binding domain may be encoded by an altered or variant form of a germ line gene with one or more nucleotide alterations (addition, deletion, substitution and/or insertion), e.g. about or less than about 25, 20, 15, 10 or 5 alterations, 4, 3, 2 or 1, which may be in one or more frameworks and/or CDR's.

The binding domain may comprise part or all of a VH domain having any amino acid sequence shown in Figure 1 (a) or a functionally equivalent variant form of the said amino acid sequence.

In particular, the binding domain may comprise one or more CDR (complementarity determining region) with an amino acid sequence identified in Figure 1 (a) as a CDR1, CDR2 or CDR3. In a preferred embodiment, the binding domain comprises a CDR3 sequence shown in Figure 1(a). Functionally equivalent variant forms of the CDRs are encompassed by the present invention, in particular variants which differ from the CDR sequences shown by addition, deletion, substitution or insertion of one or more amino acids and which retain ability to bind CEA and optionally one or more of the preferred characteristics for specific binding members of the present invention as disclosed herein. Particularly preferred variant sequences of CEA6 VH are shown in Figure 2. In a preferred embodiment of the present invention a specific binding member includes a CDR3 sequence shown in Figure 2 (or a functionally equivalent variant form thereof). The specific binding member may comprise all or part of the framework regions shown flanking and between the CDRs in Figure 2, or different framework regions including modified versions of those shown. If either of the CDR3 sequences of "HBAll" and "HBB11" (Figure 2) is employed (for example), the specific binding member may comprise an arginine (R) residue in the position shown (Figure 2) in whatever framework region is employed.

The binding domain may comprise part or all of a VL domain having any amino acid sequence shown in Figure 1 (b) or a functionally equivalent variant form of the said amino acid sequence.

In particular, the binding domain may comprise one or more CDR (complementarity determining region) with an amino acid sequence identified in Figure 1 (b) as a CDR1, CDR2 or CDR3. In a preferred embodiment, the binding domain comprises a CDR3 sequence shown in Figure 1(b). Functionally equivalent variant forms of the CDRs are encompassed by the present invention, in particular variants which differ from the CDR sequences shown by addition, deletion, substitution or insertion of one or more amino acids and which retain ability to bind CEA and optionally one or more of the preferred characteristics for specific binding members of the present invention as disclosed herein. Particularly preferred variant sequences of CEA6 VL are shown in Figure 3 and Figure 4. In a preferred embodiment of the present invention a specific binding member includes a CDR3 sequence shown in Figure 3 or Figure 4 (or a functionally equivalent variant form thereof). The specific binding member may comprise all or part of the framework regions shown flanking and between the CDRs in Figure 3 or Figure 4, or different framework regions including modified versions of those shown. Preferred framework modifications are shown in Figure 4 and these modified framework regions may or may not be used (but may be preferred for use) with one or more of the CDR sequences of "T06D4", "T06D8" or "T06D12" shown in Figure 4.

So-called "CDR-grafting" in which one or more CDR sequences of a first antibody is placed within a framework of sequences not of that antibody, e.g. of another antibody is disclosed in EP-B-0239400, which has an equivalent US patent.

Particular variants VL or VH domains may include one or more amino acid sequence alterations (addition, deletion, substitution and/or insertion), maybe less than about 20 alterations, less than about 15 alterations, less than about 10 alterations or less than about 5 alterations, 4, 3, 2 or 1, in any specific sequence provided herein. Alterations may be made in one or more framework regions and/or one or more CDR's.

Particular VH and VL variants according to the present invention are as follows: a VH domain comprising the sequence shown for CEA2 VH in Figure 1(a) save for the deletion of Ser53 in the sequence shown; a VH domain comprising the sequence shown for CEA3 VH in Figure 1(a) save for deletion of Gly53 in the sequence shown; a VL domain comprising the sequence shown for CEA6 in Figure 1(b) save for substitution of serine for Thr10 (which may be encoded by ACC at codon 10 in the underlying coding sequence) in the sequence shown; a VL domain comprising the VL sequence shown for any of CEA6, T06D10, HBA11, HBB11 and HBB6 in Figure 2 save for substitution of Val for Gln5 and GluAlaLeu for AlaGluVal at residues 9 to 11 in the sequence shown; a VL domain comprising the VL sequence shown for any of CEA6, LOB1C, LOE17, LOSC2 T06D4, TO6D8 and TO6D12 in Figure 3 or Figure 4 save for substitution of Met for Leu33 in the sequence shown; amino acid sequence variants of the foregoing variants with the specified alterations to the sequences shown.

A specific binding member according to the invention may be one which competes with any specific binding member which binds CEA and comprises part of all of any of the sequences shown in Figure 1 (a), Figure 1 (b), Figure 2, Figure 3 and Figure 4 for binding to CEA, particularly any specific binding member whose sequence is specifically disclosed herein, including variants thereof. For example, such a specific binding member may compete with TO6D11 or CEA6 for preferential binding to the A3-B3 domain of CEA, or compete with CEA1 for binding to a carbohydrate epitope of CEA. Competition between binding members may be assayed easily *in vitro,* for example by tagging a specific reporter molecule to one binding member which can be detected in the presence of other untagged binding member(s), to enable identification of specific binding members which bind the same epitope or an overlapping epitope.

In one aspect, a specific binding member according to the present invention binds a peptide including the amino acid sequence
(i) Pro Ala Ala Tyr Leu Trp Trp Val Asp Ser, or
(ii) Pro Pro Ala Tyr Leu Tyr Trp Arg Ser Ser.
(CEA6 is an embodiment of such a specific binding member.) In testing for this, a peptide with this sequence plus one or more amino acids at either end, for instance CGG at the N-terminus, may be used. Specific binding members according to the present invention may be such that their binding for CEA is inhibited by a peptide with or including either of the sequences (i) or (ii). In testing for this, a peptide with either sequence plus one or more amino acids, such as CGG at the N-terminus, may be used.

Specific binding members which bind either of the peptides (i) and (ii) may be isolated for example from a phage display library by panning with the peptide(s).

Specific binding members according to the invention may be provided in isolated and/or purified form.

The present invention provides the use of a specific binding member as above to use as a diagnostic reagent for forms of human cancer e.g. adenocarcinoma of colon, lung or breast.

The specific binding member for CEA may be used as an imaging agent which may be used to specifically demonstrate the presence and location of CEA-expressing tumours. The present invention provides a method of determining the presence of a CEA-expressing cell or tumour, the method comprising contacting cells with a specific binding member as provided and determining the binding of the specific binding member to the cells. The method may be performed *in vivo,* or *in vitro* on a test sample of cells removed from the body.

The present invention provides a method comprising causing or allowing binding of a specific binding member as as provided herein to human CEA. Such binding may take place *in vitro* or *in vivo*. If the binding is in *vivo,* the method may comprise administration of the specific binding member to a mammal, one or more individuals. As demonstrated experimentally herein, specific binding members according to the invention bind human CEA on xenografted tumours in mice, providing a useful experimental model for study for research and development purposes of the specific binding members and their properties.

The reactivities of antibodies on a cell sample may be determined by any appropriate means. Tagging with individual reporter molecules is one possibility. The reporter molecules may directly or indirectly generate detectable, and preferably measurable, signals. The linkage of reporter molecules may be directly or indirectly, covalently, eg via a peptide bond or non-covalently. Linkage via a peptide bond may be as a result of recombinant expression of a gene fusion encoding antibody and reporter molecule.

One favoured mode is by covalent linkage of each antibody with an individual fluorochrome, phosphor or laser dye with spectrally isolated absorption or emission characteristics. Suitable fluorochromes include fluorescein, rhodamine, phycoerythrin and Texas Red. Suitable chromogenic dyes include diaminobenzidine.

Other reporters include macromolecular colloidal particles or particulate material such as latex beads that are coloured, magnetic or paramagnetic, and biologically or chemically active agents that can directly or indirectly cause detectable signals to be visually observed, electronically detected or otherwise recorded. These molecules may be enzymes which catalyse reactions that develop or change colours or cause changes in electrical properties, for example. They may be molecularly excitable, such that electronic transitions between energy states result in characteristic spectral absorptions or emissions. They may include chemical entities used in conjunction with biosensors. Biotin/avidin or biotin/streptavidin and alkaline phosphatase detection systems may be employed.

The mode of determining binding is not a feature of the present invention and those skilled in the art are able to choose a suitable mode according to their preference and general knowledge.

The signals generated by individual antibody-reporter conjugates may be used to derive quantifiable absolute or relative data of the relevant antibody binding in cell samples (normal and test). In addition, a general nuclear stain such as propidium iodide may be used to enumerate the total cell population in a sample, allowing the provision of quantitative ratios of individual cell populations relative to the total cells. When a radionucleotide such as ¹²⁵I, ¹¹¹In or ^{99m}Tc is attached to an antibody, if that antibody localises preferentially in tumour rather than normal tissues, the presence of radiolabel in tumour tissue can be detected and quantitated using a gamma camera. The quality of the tumour image obtained is directly correlated to the signal:noise ratio. A review of cancer imaging with CEA antibodies is provided by Goldenberg D.M. *ibid.*

Experimental use of ¹²⁵I and ^{99m}Tc is exemplified herein.

The present invention also provides for the use of a specific binding member as above to use as a therapeutic reagent, for example when coupled, bound or engineered as a fusion protein to possess an effector function. A specific binding member according to the present invention may be used to target a toxin, radioactivity, T-cells, killer cells or other molecules to a tumour expressing CEA.

Accordingly, further aspects of the invention provide methods of treatment comprising administration of a specific binding member as provided, pharmaceutical compositions comprising such a specific binding member, and use of such a specific binding member in the manufacture of a medicament for administration, for example in a method of making a medicament or pharmaceutical composition comprising formulating the specific binding member with a pharmaceutically acceptable excipient.

In accordance with the present invention, compositions provided may be administered to individuals. Administration is preferably in a "therapeutically effective amount", this being sufficient to show benefit to a patient. Such benefit may be at least amelioration of at least one symptom. The actual amount administered, and rate and time-course of administration, will depend on the nature and severity of what is being treated. Prescription of treatment, eg decisions on dosage etc, is within the responsibility of general practioners and other medical doctors. Appropriate doses of antibody are well known in the art; see Ledermann J.A. et al. (1991) Int J. Cancer 47: 659-664; Bagshawe K.D. et al. (1991) Antibody, Immunoconjugates and Radiopharmaceuticals 4: 915-922.

A composition may be administered alone or in combination with other treatments, either simultaneously or sequentially dependent upon the condition to be treated.

Pharmaceutical compositions according to the present invention, and for use in accordance with the present invention, may comprise, in addition to active ingredient, a pharmaceutically acceptable excipient, carrier, buffer, stabiliser or other materials well known to those skilled in the art. Such materials should be non-toxic and should not interfere with the efficacy of the active ingredient. The precise nature of the carrier or other material will depend on the route of administration, which may be oral, or by injection, e.g. intravenous.

Pharmaceutical compositions for oral administration may be in tablet, capsule, powder or liquid form. A tablet may comprise a solid carrier such as gelatin or an adjuvant. Liquid pharmaceutical compositions generally comprise a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous, injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

A specific binding member according to the present invention may be made by expression from encoding nucleic acid. Nucleic acid encoding any specific binding member as provided itself forms an aspect of the present invention, as does a method of production of the specific binding member which method comprises expression from encoding nucleic acid therefor. Expression may conveniently be achieved by culturing under appropriate conditions recombinant host cells containing the nucleic acid. Following production by expression a specific binding member may be isolated and/or purified using any suitable technique, then used as appropriate, e.g. in formulation of a composition which may comprise at least one other component.

The nucleic acid may encode any of the amino acid sequences shown in Figure 1a and Figure 1(b), or any functionally equivalent form. The nucleotide sequences employed may be any of those shown in Figure 1(a) or Figure 1(b), or may be a variant, allele or derivative thereof. Changes may be made at the nucleotide level by addition, substitution, deletion or insertion of one or more nucleotides, which changes may or may not be reflected at the amino acid level, dependent on the degeneracy of the genetic code.

Systems for cloning and expression of a polypeptide in a variety of different host cells are well known. Suitable host cells include bacteria, mammalian cells, yeast and baculovirus systems. Mammalian cell lines available in the art for expression of a heterologous polypeptide include Chinese hamster ovary cells, HeLa cells, baby hamster kidney cells and many others. A common, preferred bacterial host is *E. coli.*

The expression of antibodies and antibody fragments in prokaryotic cells such as *E. coli* is well established in the art. For a review, see for example Plückthun, A. Bio/Technology 9: 545-551 (1991). Expression in eukaryotic cells in culture is also available to those skilled in the art as an option for production of a specific binding member, see for recent reviews, for example Reff, M.E. (1993) Curr. Opinion Biotech. 4: 573-576; Trill J.J. et al. (1995) Curr. Opinion Biotech 6: 553-560.

Suitable vectors can be chosen or constructed, containing appropriate regulatory sequences, including promoter sequences, terminator sequences, polyadenylation sequences, enhancer sequences, marker genes and other sequences as appropriate. Vectors may be plasmids, viral e.g. 'phage, or phagemid, as appropriate. For further details see, for example, *Molecular Cloning: a Laboratory Manual:* 2nd edition, Sambrook et al., 1989, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in *Short Protocols in Molecular Biology,* Second Edition, Ausubel et al. eds., John Wiley & Sons, 1992. The disclosures of Sambrook et al. and Ausubel et al. are incorporated herein by reference.

Thus, a further aspect of the present invention provides a host cell containing nucleic acid as disclosed herein. A still further aspect provides a method comprising introducing such nucleic acid into a host cell. The introduction may employ any available technique. For eukaryotic cells, suitable techniques may include calcium phosphate transfection, DEAE-Dextran, electroporation, liposome-mediated transfection and transduction using retrovirus or other virus, e.g. vaccinia or, for insect cells, baculovirus. For bacterial cells, suitable techniques may include calcium chloride transformation, electroporation and transfection using bacteriophage.

The introduction may be followed by causing or allowing expression from the nucleic acid, e.g. by culturing host cells under conditions for expression of the gene.

In one embodiment, the nucleic acid of the invention is integrated into the genome (e.g. chromosome) of the host cell. Integration may be promoted by inclusion of sequences which promote recombination with the genome, in accordance with standard techniques.

Following production of a specific binding member it may be used for example in any of the manners disclosed herein, such as in the formulation of a composition such as a pharmaceutical, or a diagnostic product, such as a kit comprising in addition to the specific binding member one or more reagents for determining binding of the member to cells, as discussed.

Further aspects of the invention and embodiments will be apparent to those skilled in the art. In order that the present invention is fully understood, the following examples are provided by way of exemplification only and not by way of limitation. Reference is made to the following figures:

**Figure 1** shows nucleotide and amino acid sequences of (Figure 1a) the VH genes and (Figure 1b) the VL genes of antibodies specific for CEA.

**Figure 1(a)**: Sequence alignment of CEA-specific scFvs derived from an unimmunised human library. The top panel of the figure shows the amino acid sequences of the VH genes of clones CEA1->7 inclusive; the bottom panel shows the nucleotide sequences of the same clones. CDR = complementarity determining region.

**Figure 1(b):** Sequence alignment of CEA-specific scFvs derived from an unimmunised human library. The top panel of the figure shows the amino acid sequences of the VL genes of clones CEA1 -> 7 inclusive; the bottom panel shows the nucleotide sequences of the same clones. Identical sequences between clones are indicated by dots.

**Figure 2** shows the sequences of clones derived from CEA6 by mutagenesis of VH CDR3. Aligned amino acid sequences of the VH genes of CEA6 and four clones derived from mutagenesis of the heavy chain CDR3. Identical sequences between clones are indicated by dots.

**Figure 3** shows the sequences of clones derived from CEA6 by mutagenesis of VL CDR3. Aligned amino acid sequences of the VL genes of CEA6 and three clones derived from mutagenesis of the heavy chain CDR3. Identical sequences between clones are indicated by dots.

**Figure 4** shows aligned amino acid sequences of the VL gene of CEA6 and those of three clones derived from light chain shuffling. Identical sequences between clones are indicated by dots.

The most homologous germline gene of each clone is:

| | |
|---|---|
| CEA6 | L12a |
| TO6D4 | DPK9 |
| TO6D8 | DPK9 |
| TO6D12 | Hu102 |

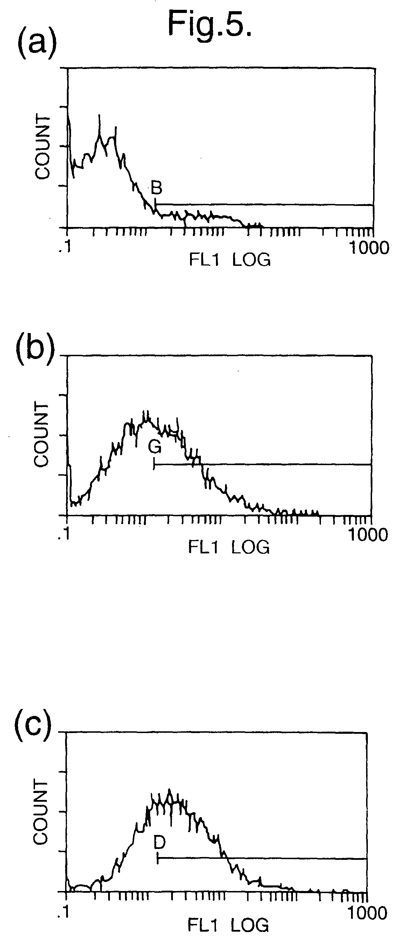

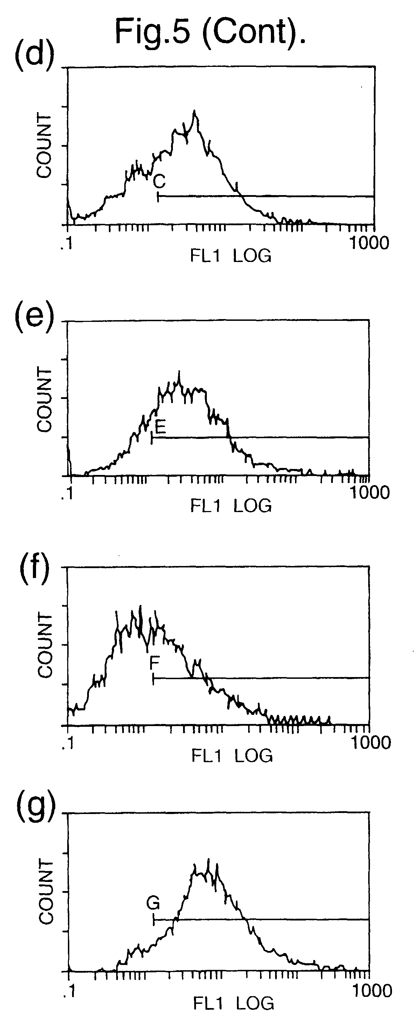

**Figure 5** shows flow cytometry analysis of CEA6 and a selection of CEA6-derived clones on CEA-expressi ng HeLa cells. The top panel (Figure 5a) shows background binding of the detecting antibody 9E10 (in the absence of added scFv) to the CEA-expressing cells. All CEA-specific clones demonstrate an approximate 10-fold shift in the number of fluorescent cells (x axis), hence demonstrating anti-CEA scFv binding to these cells. (Figure 5a - negative control; Figure 5b - T06D4; Figure 5c - T06D12; Figure 5d - HBB11; Figure 5e - T06D11; Figure 5f - T06D10; Figure 5g - CEA6)

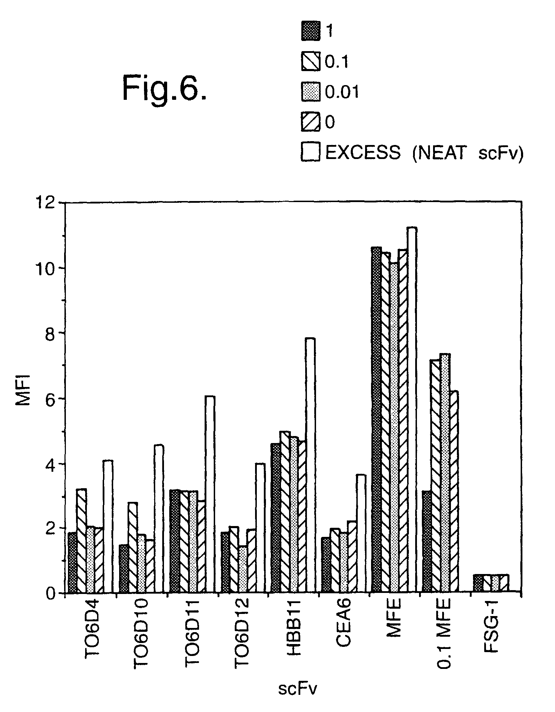

**Figure 6** shows flow cytometry analysis of CEA6 and a selection of CEA6-derived clones on CEA-expressing HeLa cells, measured in the presence of free CEA at concentrations from 0.01 to 1 *µ*g/ml. The figure shows that free CEA is unable to compete anti-CEA scFvs off the CEA-expressing HeLa cells. In contrast, a control antibody (MFE) is competed off the cells at a concentration of 1 *µ*g/ml free CEA. FSG1 = negative control. MFI = mean fluorescence intensity.

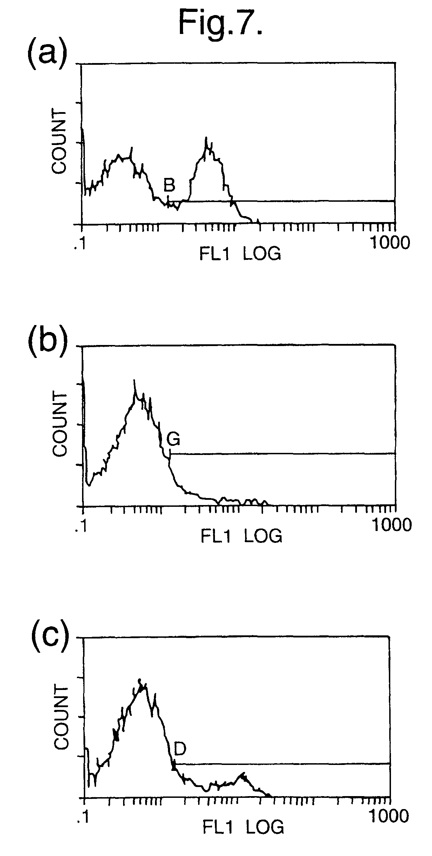

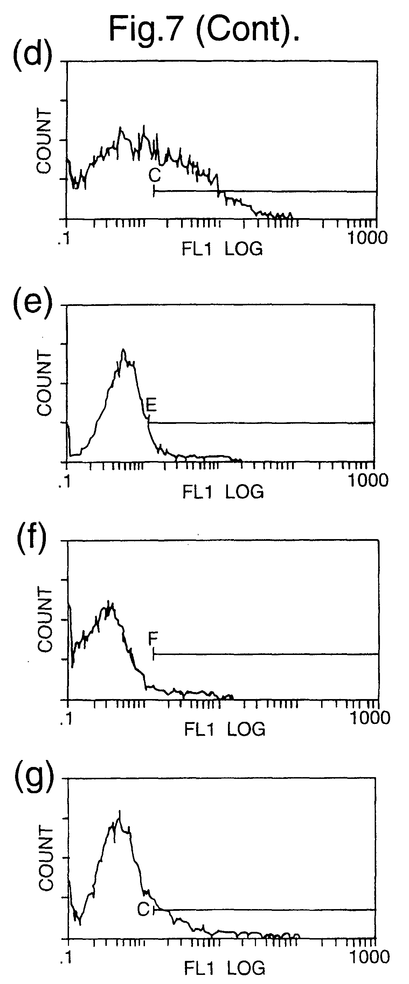

**Figure 7** shows flow cytometry analysis of CEA6 and a selection of CEA6-derived clones on CEA-negative Chang human liver cells. The top graph (Figure 7a) demonstrates that a component of CEA6 is binding to the liver cells, whereas (Figure 7b) TO6D4, (Figure 7c) TO6D12, (Figure 7e) TO6D11, (Figure 7f) TO6D10 and (Figure 7g) FSG1 (an unrelated non-CEA specific scFv - negative control) do not bind in the same way. HBB11 (Figure 7d) shows some cross-reactivity to the liver cells, as demonstrated by the broader peak of fluorescent cells.

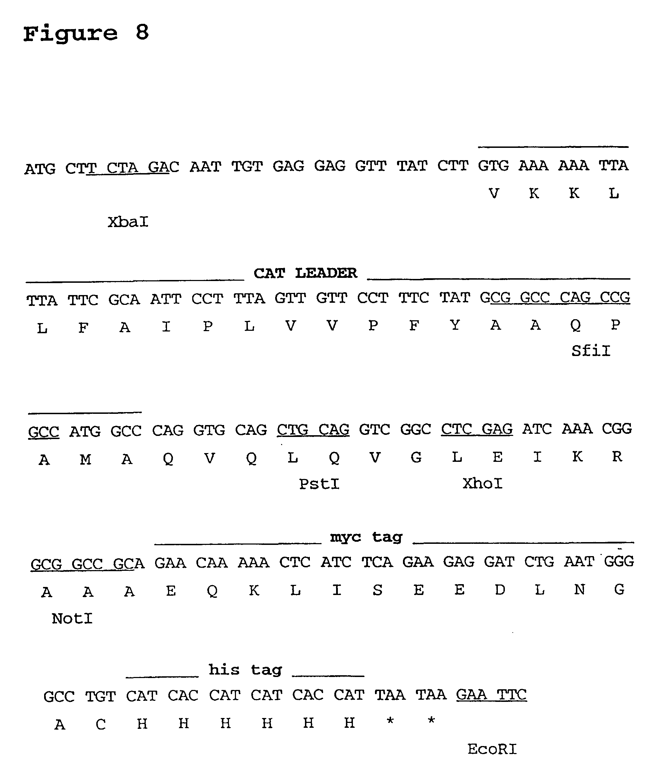

**Figure 8** shows cloning sites in the vector pUC119MCH. The vector is based upon pUC119 and carries the following features: CAT leader sequence (hybrid geneIII-pelB leader); unique NcoI and SfiI 5' cloning sites; unique NotI 3' cloning site; *myc* tag (for detection with 9E10); single cysteine residue for site-specific labelling; hexahistidine tag for IMAC purification.

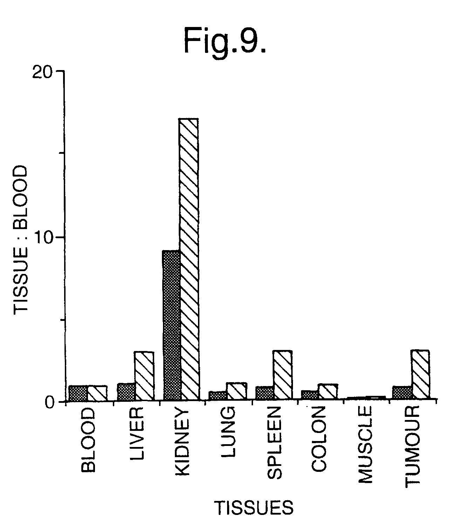

**Figure 9** shows tissue to blood ratios (for various tissues) of ^{99m}Technetium-labelled CEA6 scFv in a mouse xenograft model of human colon adenocarcinoma. Filled bars are values at 3 hours post-injection, and shaded bars are values at 24 hours post injection. After 24 hours, the ratio of incorporated radioactivity in the tumour relative to that in the blood is approximately 3.0.

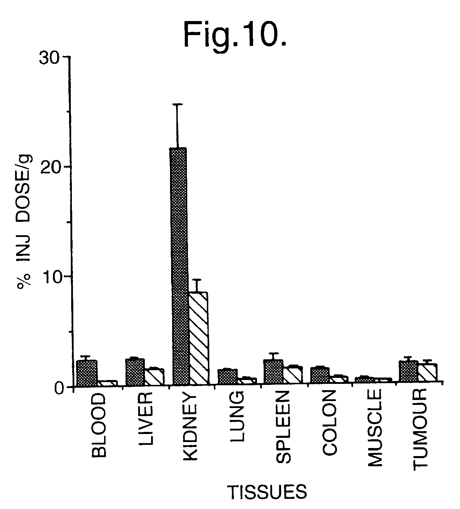

**Figure 10** shows biodistribution (various tissues) of ^{99m}Technetium-labelled CEA6 at 3 and 24 hours post-injection in the mouse xenograft model of human colon adenocarcinoma. Filled bars are values at 3 hours post-injection, and shaded bars are values at 24 hours post injection. After 24 hours, between 2 and 3% of the injected dose is found to specifically localise to the tumour.

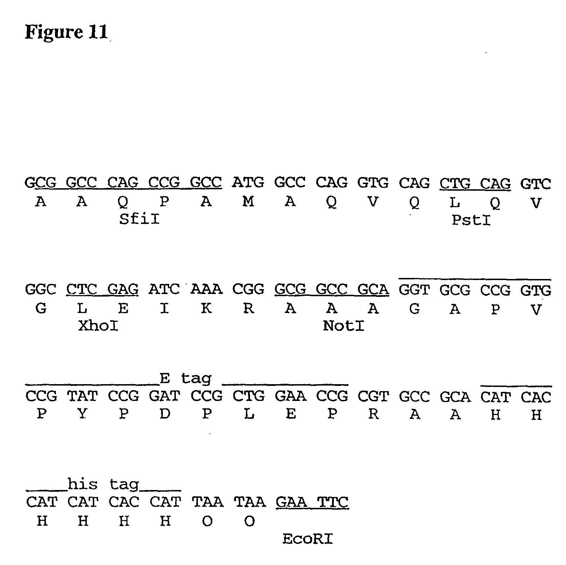

**Figure 11** shows the cloning sites in the vector pUC119EHIS. The vector is based on pUC119 and carries the following features : unique cloning sites; SfiI, PstI, XhoI, NotI; E tag for detection with ant-E tag antibodies (Pharmacia); hexahistidine tag for IMAC purification.

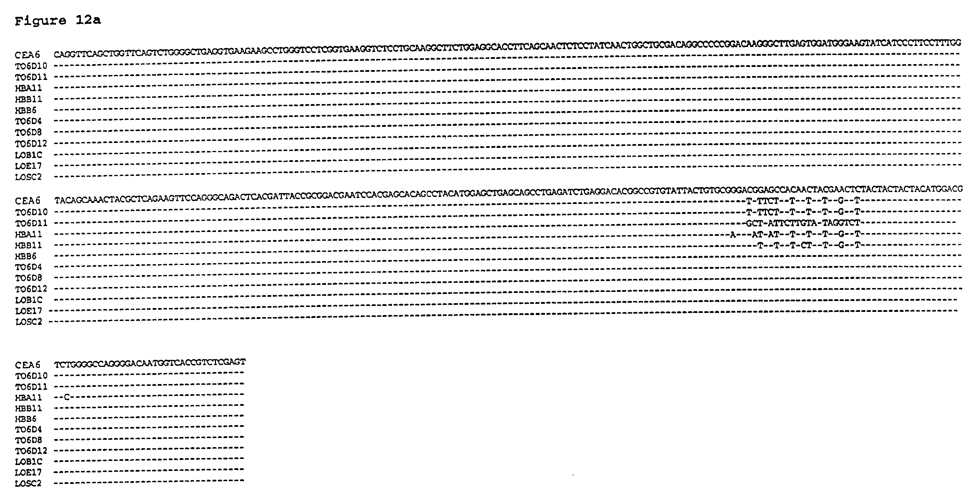

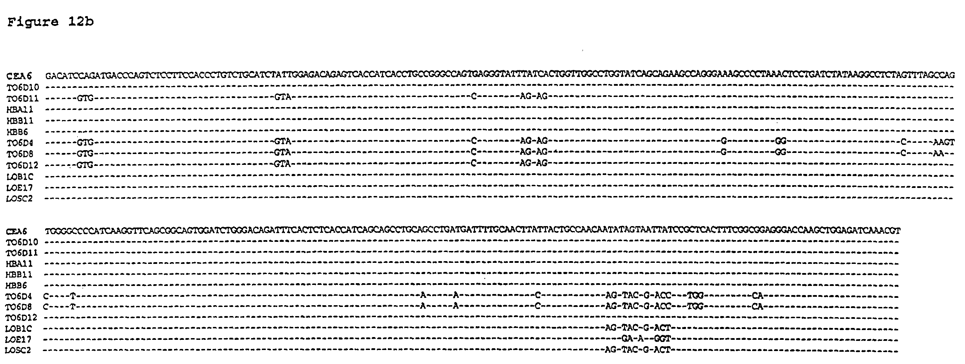

**Figure 12**: Figure 12(a) shows the nucleotide sequences of a number of VH gene segments; Figure 12(b) shows the nucleotide sequences of a number of VL gene segments.

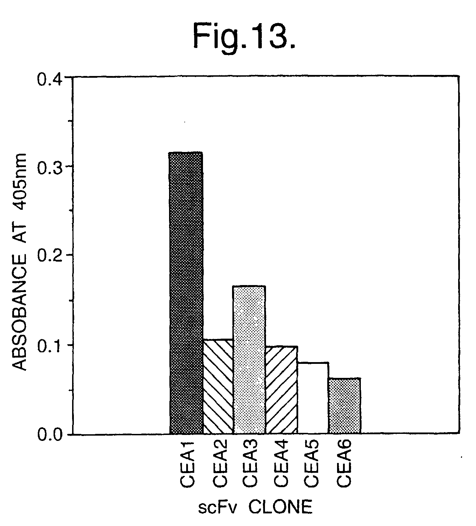

**Figure 13** shows the results of ELISA to assess whether CEA1, CEA2, CEA3, CEA4, CEA5 and CEA6 recognise K1 PSA (OD450nm v CEA clone no.).

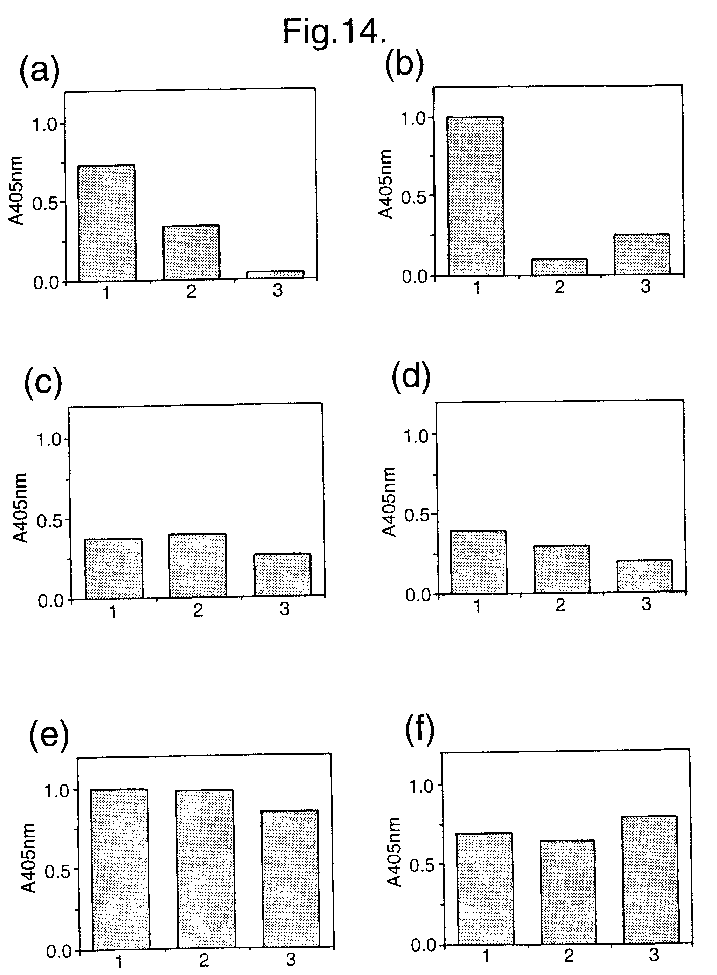

**Figure 14** shows the effect of preincubation with K1 or CA polysialic acid (PSA) on the ability of clones CEA1, CEA2, CEA3, CEA4, CEA5 and CEA6 to bind to immobilised CEA by ELISA. 1 = signal on native CEA; 2 = signal when scFv is pre-incubated with K1; 3 = signal when scFv is pre-incubated with CA. (Figure 14a - CEA1; Figure 14b - CEA2; Figure 14c - CEA3; Figure 14d - CEA4; Figure 14e - CEA5; Figure 14f - CEA6.)

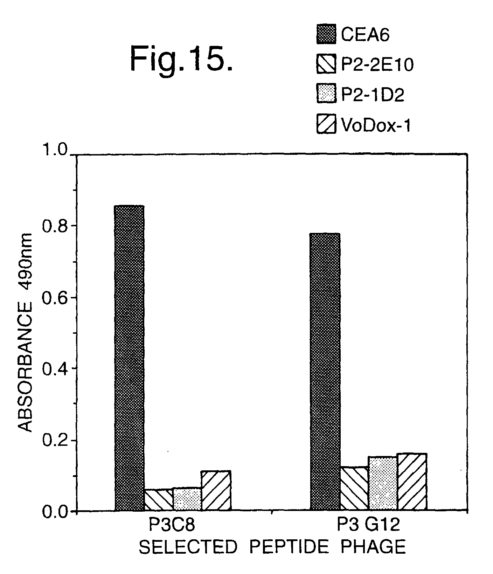

**Figure 15** shows the binding specificity of selected peptide phage for various purified scFv, plotted as the absorbance at 490nm measured by ELISA.

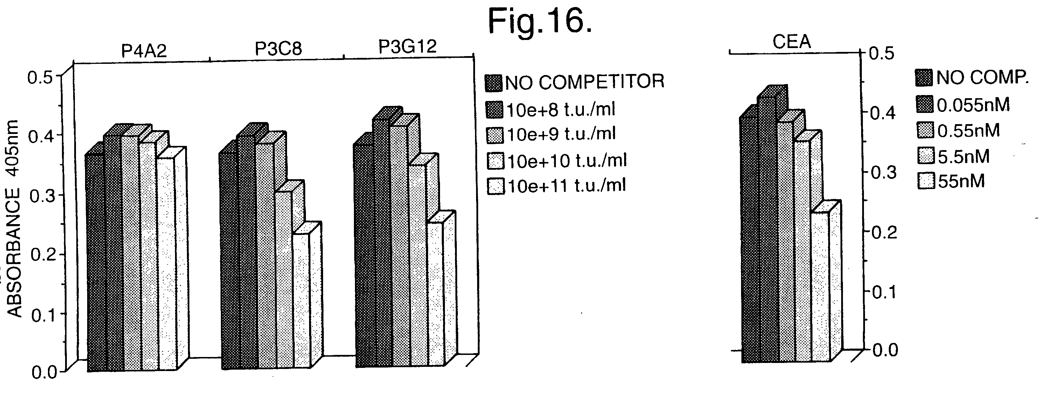

**Figure 16** shows the inhibition of binding of scFv CEA6 to CEA using selected peptide phage. Results are plotted as absorbance measured at 405nm.

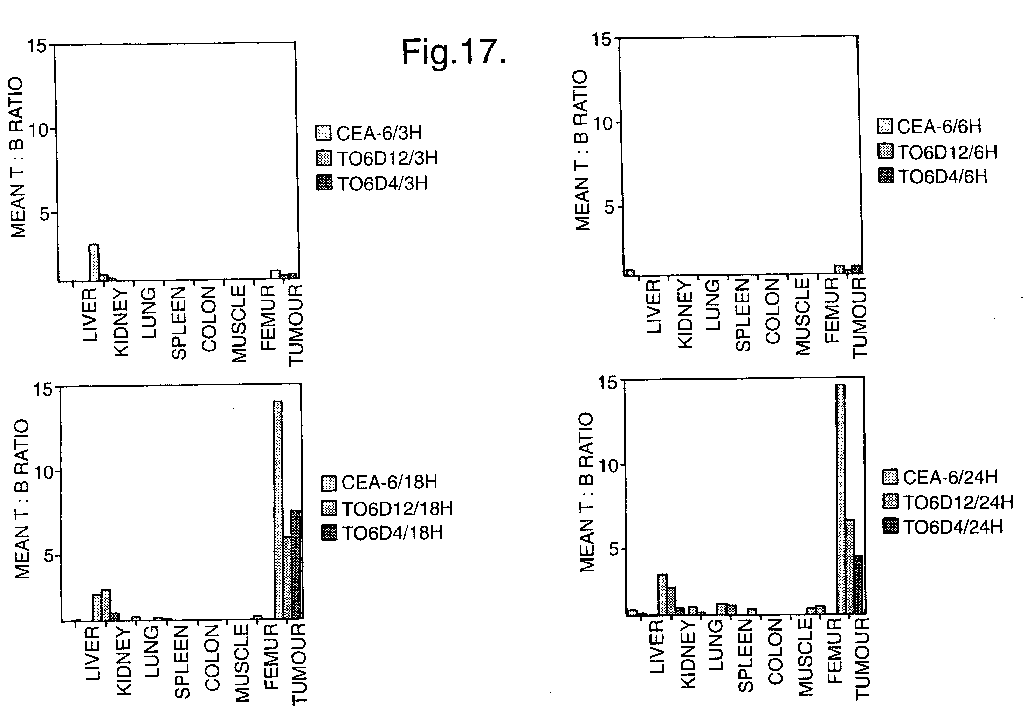

**Figure 17** shows the mean percentage of the injected dose of different ¹²⁵I-labelled scFv localised in various tissues at 3h, 6h, 18h and 24h post injection.

All documents mentioned herein are incorporated by reference.

### List of examples

**Example 1** - Isolation of antibodies specific for CEA.

**Example 2** - Affinity determination for scFv fragments binding to CEA.

**Example 3** - Demonstration of binding of antibodies specific for CEA to cell-associated CEA.

**Example 4** - Demonstration of alteration of specificity of anti-CEA antibodies for a human liver cell line.

**Example 5** - Epitope mapping of antibodies specific for CEA.

**Example 6** - *In vivo* localisation of antibodies specific for CEA to human colon adenocarcinoma xenografts.

**Example 7** - Further examination of the domain recognition of CEA6 and TO6D11.

**Example 8** - Analysis of the binding specificities of CEA1, CEA2, CEA3, CEA4 and CEA5.

**Example 9** - Immunocytochemistry of CEA1, CEA2, CEA3, CEA4, CEA5, CEA6 and affinity matured versions of CEA6.

**Example 10** - Localisation of I¹²⁵-labelled anti-CEA antibodies to human colon adenocarcinoma.

**Example 11** - Refinement of epitope mapping of antibodies specific for CEA.

**Example 12** - Analysis of tumour uptake and normal tissue biodistribution of ¹²⁵I-labelled scFv specific for CEA.

### EXAMPLE 1 - ISOLATION AND CHARACTERISATION OF ANTIBODIES BINDING TO CEA

*1. Identification and characterisation of antibodies to human CEA by selection of an unimmunised phage antibody repertoire.*

### Antibody repertoire

The following antibody repertoire was used:

Large single chain Fv library derived from lymphoid tissues including tonsil, bone marrow and peripheral blood lymphocytes.

Polyadenylated RNA was prepared from the B-cells of various lymphoid tissues of 43 non-immunised donors using the "Quickprep mRNA Kit" (Pharmacia). First-strand cDNA was synthesized from mRNA using a "First-strand cDNA synthesis" kit (Pharmacia) using random hexamers to prime synthesis. V-genes were amplified using family-specific primers for VH, Vk and Vl genes as previously described (Marks et al. (1991) J. Mol. Biol. **222**: 581-597) and subsequently recombined together with the (Gly4, Ser)3 scFv linker by PCR assembly. The VH-linker-VL antibody constructs were cloned into the Sfi I and Not I sites of the phagemid vector, pCANTAB 6. Ligation, electroporation and plating out of the cells was as described previously (Marks et al, supra). The library was made ca. 1000x larger than that described previously by bulking up the amounts of vector and insert used and by performing multiple electroporations. This generated a scFv repertoire that was calculated to have ca. 6.0 x 10⁹ individual recombinants which by Bst NI fingerprinting were shown to be extremely diverse.

### a. Induction of phage antibody library

The phage antibody repertoire above was selected for antibodies to CEA. The repertoire was treated as follows in order to rescue phagemid particles. 500 ml prewarmed (37 °C) 2YTAG (2YT media supplemented with 100 *µ*g/ml ampicillin and 2 % glucose) in a 2 l conical flask was inoculated with approximately 3 x 10¹⁰ cells from a glycerol stock (-70°C) culture of the library. The culture was grown at 37°C with good aeration until the OD600nm reached 0.7 (approximately 2 hours). M13K07 helper phage (Stratagene) was added to the culture to a multiplicity of infection (moi) of approximately 10 (assuming that an OD600nm of 1 is equivalent to 5 x 10⁸ cells per ml of culture). The culture was incubated stationary at 37°C for 15 minutes followed by 45 minutes with light aeration (200 rpm) at the same temperature. The culture was centrifuged and the supernatant drained from the cell pellet. The cells were resuspended in 500 ml 2YTAK (2YT media supplemented with 100 *µ*g/ml ampicillin and 50 *µ*g/ml kanamycin), and the culture incubated overnight at 30°C with good aeration (300 rpm). Phage particles were purified and concentrated by three polyethylene glycol (PEG) precipitations (Sambrook, J., Fritsch, E.F., & Maniatis, T. (1990). Molecular Cloning - A Laboratory Manual. Cold Spring Harbour, New York) and resuspended in PBS to 10¹² transducing units (tu)/ml (ampicillin resistant clones).

### b. Panning of phage antibody library on CEA

Phage induced from the repertoire were panned on CEA. A 75mm x 12mm immuno tube (Nunc; Maxisorp) was coated with 1 ml of recombinant human CEA (20ug/ml, Genzyme) in PBS overnight at 37 °C. After washing 3 times with PBS, the tube was filled with 3%MPBS (3 % 'Marvel' skimmed milk powder, 1x PBS) and incubated for 2 hours at 37°C for blocking. The wash was repeated, phagemid particles (10¹³ tu) in 1 ml of 3% MPBS were added and the tube incubated stationary at 37°C for 1 hour. The tube was washed 20 times with PBST (0.1%), then 20 times with PBS. Bound phage particles were eluted from the tube by adding 1 ml of 100mM-triethylamine, and incubating the tube stationary at room temperature for 10 minutes. The eluted material was immediately neutralised by pipetting into a tube containing 0.5 ml 1M-Tris.HCl (pH7.4). Phage were stored at 4°C. 0.75 ml of the eluted phage were used to infect 10 ml of logarithmically growing E. coli TG1 (Gibson, T.J. (1984). PhD thesis. University of Cambridge, UK.). Infected cells were grown for 1 hour at 37°C with light aeration in 2YT broth, and then plated on 2YTAG medium in 243mm x 243mm dishes (Nunc). Plates were incubated overnight at 30 °C. Colonies were scraped off the plates into 10 ml of 2YT broth and 15 % (v/v) glycerol added for storage at -70°C.

Glycerol stock cultures from the first round of panning the repertoire on CEA were rescued using helper phage to derive phagemid particles for the second round of panning. 250 *µ*l of glycerol stock was used to inoculate 50 ml 2YTAG broth, and incubated in a 250 mL conical flask at 37°C with good aeration until the OD600nM reached 0.7 (approximately 2 hours). M13K07 helper phage (moi=10) was added to the culture which was then incubated stationary at 37°C for 15 minutes followed by 45 minutes with light aeration (200 rpm) at the same temperature. The culture was centrifuged and the supernatant drained from the cell pellet. The cells were resuspended in 50 ml prewarmed 2YTAK, and the culture incubated overnight at 30°C with good aeration. Phage particles were purified and concentrated by PEG precipitation (Sambrook et al., 1990) and resuspended in PBS to 10¹³ tu/ml.

Phage induced from the first round of panning the repertoire was selected a second time as described above. The process of phage growth and panning was repeated over a third and a fourth round of selection.

### c. Growth of single selected clones for immunoassay

Individual colonies from the third and fourth round selections were used to inoculate 100 *µ*l 2YTAG into individual wells of 96 well tissue culture plates (Corning). Plates were incubated at 30°C overnight with moderate shaking (200 rpm). Glycerol to 15 % was added to each well and these master plates stored at -70°C until ready for analysis.

### d. ELISA to identify anti-CEA scFv

Clones specific for CEA were identified by ELISA, using scFv displayed on phage or soluble scFv.

### i. Phage ELISA

Cells from the master plates were used to inoculate fresh 96 well tissue culture plates containing 100 *µ*l 2YTAG per well. These plates were incubated at 37°C for 6-8 hours or until the cells in the wells were growing logarithmically (OD600 0.2-1.0). M13K07 was added to each well to an moi of 10 and incubated stationary for 15 min then 45 min with gentle shaking (100 rpm), both at 37 °C. The plates were centrifuged at 2000 rpm for 10 min and the supernatant removed. Each cell pellet was resuspended in 100 *µ*l 2YTAK and incubated at 30°C overnight.

Each plate was centrifuged at 2000 rpm and the 100 *µ* l supernatant from each well recovered and blocked in 20 *µ* l 18%M6PBS (18 % skimmed milk powder, 6 x PBS), stationary at room temperature for 1 hour. Meanwhile, flexible microtitre plates which had been coated overnight stationary at 37°C with either 100 *µ* l 0.5 *µ*g/ml CEA in PBS or 100 *µ*l PBS alone (giving an uncoated control plate), were washed 3 times in PBS and blocked for 2 h stationary at room temperature in 3MPBS. These plates were then washed three times with PBS and 50 *µ*l preblocked phage added to each well of both the CEA-coated or uncoated plate. The plates were incubated stationary at 37°C for 1 h after which the phage were poured off. The plates were washed by incubating for 2 min in PBST three times followed by incubating for 2min in PBS three times, all at room temperature.

To each well of both the CEA-coated and the uncoated plate, 50 *µ*l of a 1 in 10 000 dilution of sheep anti-fd antibody (Pharmacia) in 3MPBS was added and the plates incubated at 37°C stationary for 1 h. Each plate was washed as described above and 50 *µ*l of a 1 in 5 000 dilution donkey anti-sheep alkaline phosphatase conjugate (Sigma) in 3MPBS added and incubated stationary at 37°C for 1 h. Plates were washed as described as above followed by two rinses in 0.9% NaCl. Alkaline phosphatase activity was visualised using either the chromagenic substrate pNPP (Sigma) or the Ampak system (Dako). The absorbance signal generated by each clone was assessed by measuring the optical density at either 405 nm (pNPP) or 492 nm (Ampak) using a microtitre plate reader. Clones were chosen for further analysis if the ELISA signal generated on the CEA-coated plate was at least double that on the uncoated plate.

### ii. Soluble ELISA

Cells from the master plates were used to inoculate fresh 96 well tissue culture plates containing 100 *µ*l 2YTAG per well. These plates were incubated at 30°C for 8 hours then centrifuged at 2000 rpm for 10 min and the supernatant removed. Each cell pellet was resuspended in 100 *µ*l 2YTA containing 1mM IPTG and incubated at 30°C overnight.

Each plate was centrifuged at 2000 rpm and the 100 *µ* l supernatant from each well recovered and blocked in 20 *µ* l 18%M6PBS stationary at room temperature for 1 hour. Meanwhile, flexible microtitre plates which had been blocked overnight stationary at 37°C with either 100 *µ*l 0.5 *µ*g/ml CEA in PBS or 100 *µ*l PBS alone, were washed 3 times in PBS and blocked for 2 h stationary at 37°C in 3MPBS. These plates were then washed three times with PBS and 50 *µ*l preblocked soluble scFv added to each well of both the CEA-coated or uncoated plate. The plates were incubated stationary at 37°C for 1 h after which the scFv solutions were poured off. The plates were washed by incubating for 2 min in PBST three times followed by incubating for 2min in PBS three times, all at room temperature.

To each well of both the CEA-coated and the uncoated plate, 100 *µ*l of a 1 in 200 dilution of the anti-myc tag murine antibody 9E10 (Munro, S. & Pelham, H.R.B. (1986)Cell 46, 291-300) in 3MPBS was added and the plates incubated at 37°C stationary for 1 h. Each plate was washed as described above and 100 *µ*l of a 1 in 5000 dilution goat anti-mouse alkaline phosphatase conjugate (Pierce) in 3MPBS added and incubated stationary at 37°C for 1 h. Plates were washed as described above followed by two rinses in 0.9% NaCl. Alkaline phosphatase activity was visualised using the chromagenic substrate pNPP (Sigma). The absorbance signal generated by each clone was assessed by measuring the optical density at 405 nm (pNPP) using a microtitre plate reader. Clones were chosen for further analysis if the ELISA signal generated on the CEA-coated plate was at least double that on the uncoated plate.

### iii. Specificity ELISA

Clones identified as binding CEA rather than an uncoated well, as described above, were further analysed for specificity. Specificity ELISA's were carried out using scFv either displayed on phage or in solution as described above, except that 5 ml of media in 50 ml Falcon tubes were inoculated with each clone and grown to generate the phage or soluble scFv used in the ELISA. Microtitre plate wells were coated with 100 *µ*l of either 0.5 *µ*g/ml CEA, 10 *µ* g/ml bovine serum albumin (BSA), 10 *µ*g/ml ovalbumin, 10 *µ*g/ml lysozyme, 10 *µ*g/ml keyhole limpet haemocyanin (KLH) or PBS (the uncoated well). After preblocking both the phage (or soluble scFv) and the microtitre plates, 50 *µ*l blocked phage (or soluble scFv) from each clone was added to a well coated with either CEA, BSA, ovalbumin, lysozyme, KLH, or an uncoated well. As above, alkaline phosphatase activity was visualised using the chromagenic substrate pNPP (Sigma). Clones were considered to be specific for CEA if the ELISA signal generated in the CEA coated well was at least five-fold greater than the signal on any of the test antigens or an uncoated well.

### e. Sequencing of CEA-Specific ScFv Antibodies

The nucleotide sequences of the CEA specific antibodies were determined by first using vector-specific primers to amplify the inserted DNA from each clone. Cells from an individual colony on a 2YTAG agar plate were used as the template for a polymerase chain reaction (PCR) amplification of the inserted DNA using the primers pUC19reverse and fdtetseq (Table 1). Amplification conditions consisted of 30 cycles of 94°C for 1 min, 55°C for 1 min and 72°C for 2min, followed by 10 min at 72°C. The PCR products were purified using a PCR Clean-up Kit (Promega) in to a final volume of 50 *µ*l H20. Between 2 and 5 *µ*l of each insert preparation was used as the template for sequencing using the Taq Dye-terminator cycle sequencing system (Applied Biosystems). The primers mycseq10 and PCR-L-Link were used to sequence the light chain of each clone and PCR-H-Link and pUC19reverse to sequence the. heavy chain (Table 1).

### f. Sequence of the Initial CEA-Specific ScFv Antibodies

Seven different CEA specific antibodies were isolated from the selections. Each clone name and its heavy and light chain germline is given below. The complete sequence of each VH and VL domain gene is given in Figure 1(a) and (b).

| CLONE | VH FAMILY | VH GERMLINE SEGMENT | VL FAMILY | VL GERMLINE SEGMENT |
|---|---|---|---|---|
| CEA1 | VH4 | DP71 | VLambda1 | DPL5/2 |
| CEA2 | VH3 | DP47 | VLambda1 | DPL5/2 |
| CEA3 | VH3 | DP47 | VLambda1 | DPL5/2 |
| CEA4 | VH3 | DP67 | VLambda3 | DPL16 |
| CEA5 | VH3 | DP32 | VLambda3 | DPL16 |
| CEA6 | VH1 | DP10 | VKappa1 | L12a |
| CEA7 | VH1 | DP10 | VKappa1 | L12a |

### 2. Affinity Maturation of the Initial CEA-Specific ScFv Antibodies

### a. CDR3 'Spiking' of the CEA-Specific ScFv Antibody CEA6

### i. Construction of VH CDR3 'spiked' repertoire

A 63 mer mutagenic oligonucleotide primer, CEA6HCDOP, was first synthesized (see Table 1). This primer allowed spiking of 7 residues of the CEA6 VH CDR3 using a parsimonious mutagenesis strategy (Ballint and Larrick (1993) Gene **137**: 109-118. The CEA6 heavy chain was amplified by PCR using the primers LMB3 and CEA6HCDOP. Amplification conditions consisted of 25 cycles of 94°C for 1 min, 55°C for 1 min and 72°C for 1min, followed by 10 min at 72 °C. The PCR product was separated through a 1% agarose-TAE gel, the band representing the amplified VH excised, and eluted from the agarose gel using the Geneclean Kit (Bio 101).

The parental CEA6 light chain was amplified by PCR using the primers fdtetseq and CEA6JH (Table 1). Amplification conditions consisted of 25 cycles of 94°C for 1 min, 55°C for 1 min and 72°C for 1min, followed by 10 min at 72°C. The PCR product was separated through a 1% agarose-TAE gel, the band representing the amplified VL excised, and eluted from the agarose gel using the Geneclean Kit (Bio 101).

Approximately 50 ng amplified 'spiked' CEA6 heavy chain and 50 ng of amplified parental CEA6 light chain were combined. This was used in an assembly amplification after the addition of reaction buffer to 1X, dNTP's to 200 nM and 5 units Taq polymerase. Amplification conditions consisted of 7 cycles of 94°C for 1 min, 65°C for 4 min. Five *µ*l of each assembly was used as the template in a 'pull-through' amplification with the primers fdtetseq and LMB3. Amplification conditions consisted of 25 cycles of 94°C for 1 min, 55°C for 2 min and 72°C for 1min, followed by 10 min at 72°C.

The pull-through amplification product was separated through 1% agarose-TAE and the band representing the pull-through 'spiked' VH -VL excised and eluted using the Geneclean Kit. This was digested with the restriction endonucleases Sfi I and Not I (NEB) and ligated (Amersham ligation system) into the phagemid vector pCantab 6, previously digested with Sfi I and Not I. The ligation product was used to transform electrocompetent TG1 cells, plated out on 2YTAG plates and incubated overnight at 30°C. Approximately 1.1 x 10⁷ individual clones were generated from this 'spiking' of the CEA6 VH CDR3.

### ii. Selection of CEA6 VH CDR3-spiked repertoire

The CEA6 VH CDR3-spiked repertoire was selected for CEA-specific antibodies. Phagemid particles were recovered from the repertoire as described earlier for the initial library. Recovered phage were preblocked for 1 h in a final volume of 100 *µ*l 3MPBS. Approximately 10¹¹ tu phage were used in the first round selection and between 10⁹ and 10¹⁰ for subsequent selections. For the first round selections, biotinylated CEA to a final concentration of 10 nM was added to the preblocked phage and incubated stationary at 37°C for 1h. CEA was biotinylated using NHS-SS biotin (Pearce) according to the manufacturer's instructions at a molar ratio of 10:1 biotin:CEA.

For each selection, 100 *µ*l Dynabeads suspension (Dynal) was separated on a magnet and the beads recovered and preblocked for 2 h in 1 ml 3MPBS. The beads were recovered on a magnet and resuspended in the phagemid/biotinylated CEA mixture and incubated at room temperature for 15 min while being turned end-over-end. The beads were captured on a magnet and washed three times with PBST followed by three washes in PBS. After each wash, the beads were captured on a magnet and resuspended in the next wash. Finally, half of the beads were resuspended in 10 *µ*l dithiothreitol 50 mM DTT (the other half of the beads stored at 4°C as a back-up) and incubated at room temperature for 5 min. The whole bead suspension was then used to infect 5 ml logarithmically-growing TG1 cells. This was incubated at 37°C, stationary for 15 min then with moderate shaking for 45 min, plated on 2YTAG plates and incubated overnight at 30 °C.

Colonies were scraped off the plates into 10 ml of 2YT broth and 15 % (v/v) glycerol added for storage at -70 °C.

### iii. Identification of CEA-Specific ScFv Antibodies from the CEA6 VH-Spiked Repertoire

ScFv antibodies specific to CEA were identified by both phage and soluble ELISA, and sequenced, as described earlier. Four new CEA-specific scFv antibodies were identified. All had the CEA6 light. chain sequence (L12a), described earlier and changes in one or more of the 7 targeted spiked residues of the VH. The sequences are given in Figure 2.

### iv Construction of CEA6 VL/VH CDR3-'spiked' repertoire

A 65 mer mutagenic oligonucleotide primer, CEA6LCDOP, was first synthesized (see Table 1). This primer allowed spiking of 4 residues of the CEA6 VL CDR3 using a parsimonious mutagenesis strategy (Ballint and Larrick, supra). The CEA6 light chain was amplified by PCR using the primers CEA6JH and CEA6LCDOP. Amplification conditions consisted of 25 cycles of 94°C for 1 min, 55°C for 1 min and 72°C for 1min, followed by 10 min at 72°C. The PCR product was separated through a 1% agarose-TAE gel, the band representing the amplified VL excised, and eluted from the agarose gel using the Geneclean Kit (Bio 101).

A population of CEA6-derived heavy chains from the 10nM biotin-CEA selection described above was amplified by PCR using the primers PCRHLINK and LMB3 (Table 1). Amplification conditions consisted of 25 cycles of 94°C for 1 min, 55°C for 1 min and 72°C for 1min, followed by 10 min at 72°C. The PCR product was separated through a 1% agarose-TAE gel, the band representing the amplified VH population excised, and eluted from the agarose gel using the Geneclean Kit (Bio 101).

Approximately 50 ng amplified 'spiked' CEA6 light chain and 50 ng of the amplified parental CEA6 heavy chain population were combined. This was used in an assembly amplification after the addition of reaction buffer to 1X, dNTP's to 200 nM and 5 units Taq polymerase. Amplification conditions consisted of 7 cycles of 94°C for 1 min, 65°C for 4 min. Five *µ* l of each assembly was used as the template in a 'pull-through' amplification with the primers fdtetseq and LMB3. Amplification conditions consisted of 25 cycles of 94°C for 1 min, 55°C for 2 min and 72°C for 1min, followed by 10 min at 72°C.

The pull-through amplification product was separated through 1% agarose-TAE and the band representing the pull-through 'spiked' VH-VL excised and eluted using the Geneclean Kit. This was digested with the restriction endonucleases Sfi I and Not I (NEB) and ligated (Amersham ligation system) into the phagemid vector pCantab 6, previously digested with Sfi I and Not I. The ligation product was used to transform electrocompetent TG1 cells, plated out on 2YTAG plates and incubated overnight at 30 °C. Approximately 6 x 10⁶ individual clones were generated from this VL CDR3 'spiking' of the CEA6 VL CDR3.

### v. Selection of CEA6 VL/VH CDR3-spiked repertoire

The CEA6 VL/VH CDR3-spiked repertoire was selected for CEA-specific antibodies. Phagemid particles were recovered from the repertoire as described earlier for the initial library. Recovered phage were preblocked for 1 h in a final volume of 100 *µ*l 3MPBS. Approximately 10¹¹ tu phage were used in the first round selection and between 10⁹ and 10¹⁰ for subsequent selections. For the first round selections, biotinylated CEA to a final concentration of 10 nM was added to the preblocked phage and incubated stationary at 37°C for 1h.

For each selection, 100 *µ*l Dynabeads suspension (Dynal) was separated on a magnet and the beads recovered and preblocked for 2 h in 1 ml 3MPBS. The beads were recovered on a magnet and resuspended in the phagemid/biotinylated CEA mixture and incubated at room temperature for 15 min while being turned end-over-end. The beads were captured on a magnet and washed three times with PBST followed by two washes in PBS. Selection for clones with a longer off rate than that of CEA6 was then carried out. Beads were washed in PBS containing CEA at a concentration of 50nM. At various time points (15', 30', 1 hour, 3 hours and 18 hours) the phage captured on the magnetic beads were separated on a magnet and the wash solution was replaced. Finally, half of the beads were resuspended in 10 *µ*l 50 mM DTT (the other half of the beads stored at 4°C as a back-up) and incubated at room temperature for 5 min. The whole bead suspension was then used to infect 5 ml logarithmically-growing TG1 cells. This was incubated at 37 °C, stationary for 15 min then with moderate shaking for 45 min, plated on 2YTAG plates and incubated overnight at 30 °C.

### vi. Identification of CEA-Specific ScFv Antibodies from the CEA6 VH/VL-Spiked Repertoire

ScFv antibodies specific to CEA were identified by both phage and soluble ELISA, and sequenced, as described earlier. Three new CEA-specific scFv antibodies were identified. All three had the CEA6 heavy chain sequence (DP10), described earlier and changes in the 4 targeted spiked residues of the VL. The sequences are given in Figure 3.

### b. Light Chain Shuffling of the CEA-Specific ScFv Antibody CEA6

### i. Construction of Repertoire

The population of CEA6 VH CDR3-spiked clones described above was recombined with the complete repertoire of light chains derived from the PBL and tonsil-derived scFv repertoires. The CEA6 VH CDR3-spiked heavy chains were amplified by PCR using the primers PCRHLINK (Table 1) and LMB3. Amplification conditions consisted of 25 cycles of 94°C for 1 min, 55°C for 1 min and 72°C for lmin, followed by 10 min at 72 °C. The PCR product was separated through a 1% agarose-TAE gel, the band representing the amplified VH excised, and eluted from the agarose gel using the Geneclean Kit (Bio 101).

The tonsil light chains were amplified by PCR using the primers fdtetseq and PCRLLINK (Table 1). Amplification conditions consisted of 25 cycles of 94°C for 1 min, 55°C for 1 min and 72°C for 1min, followed by 10 min at 72°C. The PCR product was separated through a 1% agarose-TAE gel, the band representing the amplified VL excised, and eluted from the agarose gel using the Geneclean Kit (Bio 101).

Approximately 50 ng amplified CEA6 VH CDR3-spiked heavy chains and 50 ng of amplified tonsil-derived light chains were combined. This was used in an assembly amplification after the addition of reaction buffer to 1X, dNTP's to 200 nM and 5 units Taq polymerase. Amplification conditions consisted of 7 cycles of 94°C for 1 min, 60°C for 1 min and 72°C for 1min 30 s, followed by 10 min at 72°C. 10 *µ*l of each assembly was used as the template in a 'pull-through' amplification with the primers fdtetseq and LMB3 Amplification conditions consisted of 25 cycles of 94°C for 1 min, 60°C for 1 min and 72°C for 1min 30 s, followed by 10 min at 72°C.

The pull-through amplification product was separated through 1% agarose-TAE and the band representing the pull-through VH-VL excised and eluted using the Geneclean Kit. This was digested with the restriction endonucleases Sfi I and Not I (NEB) and ligated (Amersham ligation system) into the phagemid vector pCantab 6, previously digested with Sfi 1 and Not I. The ligation product was used to transform electrocompetent TG1 cells, plated out on 2YTAG plates and incubated overnight at 30 °C. Approximately 3 x 10⁷ individual clones were generated from the light chain-shuffle of the CEA6 VH CDR3-spiked heavy chains with the tonsil-derived light chains.

### ii. Selection of Light Chain Shuffle Repertoire

The light chain-shuffle repertoire was selected for CEA-specific antibodies with longer off rates than CEA6 exactly as described above for the CEA6 VH/VL CDR3-spiked repertoire.

### iii. Identification of CEA-Specific ScFv Antibodies from the Light Chain Shuffle Repertoire

ScFv antibodies specific to CEA were identified by both phage and soluble ELISA, and sequenced, as described earlier. Three new CEA-specific scFv antibodies were identified. All three had the CEA6 heavy chain sequence (DP10), described earlier. The sequences are summarised below and the complete sequence of each VL domain gene is given in Figure 4.

| CLONE | VH GERMLINE SEGMENT | VL ISOTYPE |
|---|---|---|
| TO6D4 | DP10 (CEA6) | VKappa |
| TO6D8 | DP10 (CEA6) | VKappa |
| TO6D12 | DP10 (CEA6) | VKappa |

### 3. Building higher affinity anti-CEA antibodies

Recombining heavy chains derived from high affinity anti-CEA scFv with light chains derived from anti -CEA scFv showing improved off-rate and reduced human liver cross-reactivity.

Antibodies derived by spiking CDR3 of the scFv antibody CEA6 bind CEA with high affinity (section 2b). To improve the chance of obtaining higher affinity antibodies it was decided to combine VHs derived from high affinity anti-CEA scFvs with VLs derived from scFv clones with longer off rates and with reduced human liver cross-reactivity (Example 4).

The heavy chain from clone TO6D10 was amplified by PCR using the primers LMB3 and PCR-H-Link (Table 1). Amplification conditions consisted of 25 cycles of 94°C for 1 min, 55°C for 1 min and 72°C for lmin, followed by 10 min at 72°C. The PCR product was separated through a 1% agarose-TAE gel, the band representing the amplified VH excised, and eluted from the agarose gel using the Geneclean Kit (Bio 101).

Light chains were separately amplified by PCR from the anti CEA-specific clonesTO6D8 and TO6D12 using the primers fdtetseq and PCRLLink (Table 1). The same PCR conditions were used as described for theVH amplification. Each VL PCR product was then separately purified through a 1% agarose-TAE gel as described above.

Approximately 50 ng amplified heavy chain and 50 ng of either of the amplified light chains were combined. These were used in assembly amplifications after the addition of reaction buffer, dNTP's to 200 nM and 5 units Taq polymerase. Amplification conditions consisted of 7 cycles of 94°C for 1 min, 55°C for 1 min and 72°C for 2 mins, followed by 10 min at 72°C. 5 *µ*l of assembly was used as the template in a 50ul 'pull-through' amplification with the primers fdtetseq and LMB3. Amplification conditions consisted of 25 cycles of 94°C for 1 min, 55°C for 1 min and 72°C for 2mins, followed by 10 min at 72°C.

The pull-through amplification products were separated through 1% agarose-TAE and the bands representing the pull-through VH-VL's excised and eluted using the Geneclean Kit. These were digested with the restriction endonucleases Sfi I and Not I (NEB) and ligated into the phagemid vector pCantab 6, previously digested with Sfi 1 and Not I, using the Amersham ligation system. The ligation products were used to transform electrocompetent TG1 cells, plated out on 2YTAG plates and incubated overnight at 30 °C.

### c. Identification of recombined clones T06D9 and TO6D11.

Clones which possessed the T06D10 heavy chain in combination with either the TO6D8 light chain (giving clone TO6D9) or the TO6D12 light chain (giving clone TO6D11) were identified by sequencing.

### EXAMPLE 2 - AFFINITY DETERMINATION FOR SCFV FRAGMENTS BINDING TO CEA

Affinities of all anti-CEA scFv's derived from the CEA6 parental clone were determined by surface plasmon resonance, while affinities of CEA1-5 were measured by binding inhibition ELISA.

### a. Affinity determination by surface plasmon resonance

The off-rates for binding to CEA of the scFv fragments described in example 1 were determined using desialylated CEA coupled to the sensor chip. 100*µ*g of CEA was resuspended in 0.1M sodium acetate buffer pH4.0 and desialylated using 1.375mU sialidase (Sigma). This was incubated for 4 hours at 37°C with occasional shaking. The desialylated CEA was then oxidised using 1 unit of galactose oxidase per 500*µ* g of CEA in 10mM phosphate buffer pH7.0. This was incubated for 2 hours at 36°C and desalted into 10mM sodium acetate buffer pH4.0 using a Centricon column. The CEA was then immobilised onto the sensor chip via aldehyde coupling. 15*µ*l EDC/NHS coupling agent (Pierce) was passed over the chip at a flow rate of 5*µ*l/min. 35 *µ* l of 5mM hydrazine in water was then passed over the chip, followed by 35*µ*l of ethanolamine. 4 *µ*l of 60*µ* g/ml treated CEA was passed over the chip at a flow rate of 2*µ*l/min followed by 40*µ*l 0.1M sodium cyanoborohydride in 0.1M acetate buffer pH4.0 at a flow rate of 5*µ*l/min. Approximately 1500RU (resonance units) of CEA was bound using this method. 5000RU and 800RU CEA chips were made using this procedure. Saturation of the chip with purified scFv (see Example 3) was demonstrated for each sample before off-rate calculations were performed. On- and off-rates were also calculated using the Bia-Evaluation software and the assumption that scFv preparations were 100% active. Results are shown in Table 2.

### b. Affinity measurement by inhibition ELISA

The affinities of CEA1 - > 5 inclusive could not be evaluated by surface plasmon resonance because these scFvs recognise carbohydrate structures removed by desialylation of CEA. Therefore their affinities were measured by binding inhibition ELISA.

Soluble scFv ELISAs were carried out as described in example 1. A dilution series of the scFv preparations in PBS was made to assess the point at which a signal of approximately 0.2 OD units came up on ELISA overnight. This concentration of scFv was then pre-incubated overnight at 4°C with native CEA at concentrations ranging from 20nM to 0.1nM. The resultant data were plotted as a Klotz plot (y axis = Maximal absorbance/(Maximal absorbance-absorbance at CEA conc. n) x axis = 1/CEA conc. n). The gradient of the plot was taken to be the dissociation constant. The results are shown in Table 3.

### EXAMPLE 3 - DEMONSTRATION OF THE BINDING OF ANTI-CEA ANTIBODIES TO CELL-ASSOCIATED CEA.

### a. CEA-expressed on the surface of HeLa cells.

For these experiments metal affinity chromotography (IMAC) purified scFv was used throughout; this was prepared as follows. Colonies were inoculated into 50ml of 2TY containing 2% glucose and 100*µ*g/ml ampicillin (2TY/G/A) and incubated overnight at 30°C. The overnight culture was then added to 500ml of 2TY/G/A and grown at 30°C in a shaking incubator for 1 hour. Cells were pelleted at 8K for 10 minutes, resuspended in 500ml 2TY containing 1mM IPTG and 100*µ*g/ml ampicillin and grown at 22°C overnight. Periplasmic preparations were made by pelleting the cells at 8K for 10 minutes in a precooled rotor (4°C). Pellets were resuspended in 25ml ice-cold 50mM Tris-HCl pH8, 20% w/v sucrose, 1mM EDTA and incubated on ice for 15 minutes. ScFv was then purified from the periplasmic preparation by IMAC using NTA-Agarose (Qiagen) according to the manufacturer's instructions.

1 X 10⁵ HeLa cells expressing CEA were incubated for 1 hour at room temperature with 5*µ*g of IMAC-purified anti-CEA scFv or 5*µ*g of control scFv specific for human foetal haemoglobin (FSG-1), made up to 100*µ*l in PBS/0.5%-w/v BSA (PBS/BSA). Cells were washed once in 10ml PBS/BSA and incubated with a mouse anti-myc antibody (9E10) at 25*µ*g/ml in 100ml PBS/BSA for 1 hour. Cells were washed in 10ml PBS/BSA and incubated with a 100 *µ*l of a 1:200 dilution of FITC-conjugated anti-mouse antibody (Sigma) in PBS/BSA. After a final wash in 10 ml PBS/BSA, cell fluorescence was measured by flow cytometry using a Coulter-EPISXL-MCL flow cytometer. 1 x 10³ fluorescence events were measured using the FL1 channel (emission below 550nM) and were plotted on a log scale against number of cells.

The results for a selection of the off-rate matured anti-CEA scFv's are shown in Figure 5.

### b. Anti-CEA scFv's preferentially bind to cell-associated CEA, rather than soluble CEA.

Flow cytometry analysis was carried out as above expect that soluble CEA was added to the HeLa cells expressing CEA before addition of the scFv. A range of concentrations from 10ng/ml up to 1µg/ml was added to the cells (Figure 6). At none of the concentrations tested did the soluble CEA inhibit binding to the cells of monoclonal antibody. Addition of soluble CEA was able to inhibit the binding of an unrelated monoclonal antibody of similar affinity to the cells. This suggests that CEA6 and affinity matured scFv thereof preferentially bind cell-associated CEA over soluble CEA.

### EXAMPLE 4 - DEMONSTRATION OF ALTERATION OF SPECIFICITY OF AFFINITY MATURED ANTI-CEA ANTIBODIES FOR A HUMAN LIVER CELL LINE.

Flow cytometry was carried out exactly as described in Example 3 part a, except that 1 x 10⁵ Chang human liver cells were incubated with the IMAC-purified anti-CEA scFv's or control scFv. 1 x 10³ fluorescence events were measured using the FL1 channel (emission below 550nm) and plotted on a log scale against number of cells. The results are shown in Figure 7.

It can be seen from Figure 7 that CEA6 is partially cross-reactive with the human liver cell line. HBA11 and HBB11 also give some cross-reactivity, whereas clones which were isolated by selections from the light chain shuffled repertoire have no observable cross-reactivity to the liver cell line in this assay. Thus it has been demonstrated that the selection protocol adopted has enriched for anti-CEA antibodies which have reduced cross-reactivity for human liver.

### EXAMPLE 5 - EPITOPE MAPPING OF ANTIBODIES SPECIFIC FOR CEA.

### a. Expression of full length CEA or CEA epitopes N, A1-B1, A2-B2, A3-B3.

CEA is comprised of an NH2 terminal domain (Domain N) of 108 amino acid residues followed by three highly homologous internal domains (A1-B1, A2-B2, A3-B3) of 178 residues each. The 23 residue C-terminal domain (Domain M) has been shown to be removed post-translationally and replaced with a glycophospholipid moiety that anchors CEA in the cell membrane. cDNA of full length CEA or epitopes N, A1-B1, A2-B2 or A3-B3 as fusion proteins with bacterial CMP-KDO synthetase (CKS) were provided by Dr J. Shively (Hass et al (1991) Cancer Res. 51: 1876-1882).

XL1-Blue cells containing the CKS-CEA genes were cultured as follows. Cultures of 2ml of 2TY containing 50*µ*g/ml ampicillin were inoculated with a single colony. Cultures were incubated for approximately 3 to 4 hours at 37°C and IPTG was added to a final concentration of 1mM. Growth was continued for an additional 5 hours, then the cells were pelleted and frozen at -70°C. Cell pellets were resuspended in 3 ml of 10mM Tris, 1mM EDTA, pH 10.0. Lysozyme (6mg) was added and the samples were placed on ice for 15 minutes. 0.3 ml of 20% Triton X-100 were added and the suspension mixed. An additional 3ml of 10mM Tris, 1mM EDTA, pH 10.0, were added. The Triton-insoluble fraction was pelleted by centrifugation and resuspended in 6 ml of 8M urea. The urea-soluble material was then dialysed against PBS (0.15M NaCl, 0.02M sodium phosphate, pH 7.2) to yield soluble protein.

### b. Epitope mapping of anti-CEA scFv's derived from CEA6

The soluble domains were coated onto ELISA plates at a concentration of 1 *µ*g/ml at 37°C overnight. Soluble anti-CEA scFv was purified using metal affinity chromatography (IMAC) as described in Example 3 part a. ELISA's were carried out as described in Example 1, except that purified scFv was used at a concentration of approximately 1 *µ*g/ml. All CEA6-derived clones bound preferentially to the A3-B3 domain.

Thus it has been demonstrated that although the specificity for human liver cells has been altered by the affinity maturation procedure this is not reflected in the CEA domain specificity of the affinity matured clones.

### c. Epitope mapping of CEA1, CEA2, CEA3, CEA4 and CEA5

These clones were tested by soluble ELISA for binding to desialylated CEA (prepared as described in Example 2). Treated or untreated CEA was coated onto an ELISA plate at 0.5*µ*g/ml and the ELISA then carried out as described in Example 1. CEA1, CEA2, CEA3, CEA4 and CEA5 all gave no detectable signal above background (0.1 OD units after 2 hours development) on the desialylated CEA whereas the signals on native CEA were all >0.4 OD units after 2 hours. This demonstrates that this set of clones recognises carbohydrate epitopes on native CEA which can be removed by sialidase treatment. None of the set of clones bound to the expressed CEA epitopes N, A1-B1, A2-B2 or A3-B3 in ELISA's. Since proteins expressed in *E. coli* are not glycosylated, this result confirms the observations with desialylated CEA.

### EXAMPLE 6 - LOCALISATION OF ANTIBODIES SPECIFIC FOR CEA TO HUMAN COLON ADENOCARCINOMA XENOGRAFTS.

It has been shown in a mouse xenograft model of human colon adenocarcinoma that radiolabelled mouse anti-CEA mAb's localise to the tumour (Pedley, et al. (1991) Int. J. Cancer **47**: 597-602). A study was set up to establish whether the anti-CEA scFv antibodies described here are capable to successfully localising to the tumour in such a model.

### a. Subcloning of scFv into a cysteine-tagged vector to allow radiolabelling

ScFv inserts of all the anti-CEA antibodies were generated by PCR using the primers LMB3 and ftdseq. Amplification conditions consisted of 25 cycles of 94°C for 1 min, 55°C for 1 min and 72°C for 1 min, followed by 10 min at 72°C. The PCR products were separated through a 1% agarose-TAE gel, the band representing the amplified scFv excised, and eluted from the agarose gel using the Geneclean Kit (Bio 101). The product was digested with the restriction endonucleases Sfi I and Not I (NEB) and ligated (Amersham ligation system) into Sfi I and Not I digested cysteine tagged vector pUC119MCH (Figure 8). The ligation product was used to transform electrocompetent TG1 cells, plated out on 2TYAG plates and incubated overnight at 30°C. Colonies were picked and the scFv sequenced to check the insert had been correctly incorporated into the pUC119MCH vector.

### b. Technetium-99m labelling of IMAC purified scFv.

Anti-CEA scFv's were purified by IMAC, as described in Example 3. These were then radiolabelled with technetium-99m as described in Pak et al (1992), Nucl. Med. Biol. 19; 699-677.

### c. Animal model.

Human LS174T xenografts were established in nude mice by subcutaneous passaging from the human colon adenocarcinoma cell line LS174T. Groups of 4 mice were taken for each time point. The mice were injected through the tail vein with 20 *µ*g of technetium-99m labelled CEA6 scFv at a specific activity of 3 mCi/mg. Mice were killed either 3 or 24 hours after injection and the biodistribution and tissue:blood ratios of the antibody were measured.

The results obtained for CEA6 scFv are shown in Figure 9 and Figure 10. The tumour : blood was calculated to be 3, the tumour : liver was 1.2 and the tumour: normal colon was 3.6 at 24 hours post injection. Thus it has been demonstrated that the anti-CEA scFv CEA6 localises to a human xenografted adenocarcinoma in a nude mouse model of the disease.

### EXAMPLE 7 - FURTHER EXAMINATION OF THE DOMAIN RECOGNITION OF CEA6 AND TO6D11.

### a. Coupling scFv's to a sensor chip via the terminal cysteine residues

Monomer preparations of CEA6 or T06D11 made in the pUC119MCH vector and hence possessing a terminal cysteine residue were coupled to a CM5 chip (Pharmacia) using a ligand thiol immobilisation method as follows. 50 *µ*l of 50mM 1-ethyl-3-(3-dimethylamino-propyl) cardodiimide-HCl (EDC) reagent (Pharmacia) and 50 *µ*l of 200mM N-Hydroxysuccinimide (NHS) reagent (Pharmacia) were mixed and passed over the chip at a flow rate of 5*µ*l/min. 20 *µ*l of 80mM 2 - (2-pyridinyldithio)ethaneaminehydrochloride (PDEA) activation solution was then passed over the chip at the same flow rate. The PDEA solution was made freshly by dissolving 4.5 mg of PDEA (Sigma) in 250 *µ*l of 0.1M borate buffer pH 8.5. 20 *µ* l of an approximately 100*µ*g/ml solution of purified monomeric scFv was made up to 50 *µ*l with PBS and 50 *µ*l of 50mM sodium formate pH4 was added to the scFv and 50 *µ*l of this passed over the chip, again at 5 *µ*l/min. 50mM l-cysteine-1M NaCl deactivating solution was prepared by dissolving 1.5 mg l-cysteine and 14 mg NaCl in 250 *µ*l 0.1M sodium formate buffer pH 4.3 and 20 *µ*l of this was injected over the chip at 5 *µ*l/min. This procedure resulted in the immobilisation of 375 Resonance Units (RU's) of TO6D11, and 354 RU's of monomeric CEA6 scFv being coupled to the chip. A control chip consisting of a known N-domain reactive scFv was also prepared by the same procedure.

### b. Preparation of purified CEA domains.

50 ml cultures of the CEA domains described in Example 5a, which had been cloned into the vector pUC119EHIS vector (Figure 11) without the bacterial CKS gene were grown overnight at 30°C in 2TY containing 2% glucose and 100*µ*g/ml ampicillin (2TYGA). These cultures were used to inoculate 500ml of 2TYGA and were grown at 30°C for a further hour. Cells were pelleted by centrifugation at 5K for 10 min and resuspended in 2TY with 1mM IPTG and 100µg/ml ampicillin which had been prewarmed to 30°C. Induction was carried out with shaking for 3 hours at 30°C and the cells then pelleted as before. Pellets were resuspended in 10 ml of 1 x TES. (0.2M Tris-HCl, 0.5mM EDTA, 0.5M sucrose) and 15ml of 0.2 X TES then added. Cells were left on ice for 30 min and cell debris then pelleted at 10K for 30min at 4°C in a Sorvall SS34 rotor. The supernatant was transferred to a 50 ml Falcon tube and 25 *µ*l of 1M MgCl₂; _{QIAGEN} added. 2ml Ni-NTA agarose (Quiagen) which had been washed in phosphate buffer (300mM NaCl, 50mM sodium phosphate pH8) was added to the supernatant and rotated at 4°C for 1 hour, The Ni-NTA agarose was then pelleted by spinning in a bench-top centrifuge at 1000 rpm for 2 min and the agarose pellet washed in twice in 20 ml phosphate buffer (300mM NaCl, 50mM sodium phosphate pH8), followed by one wash in phosphate buffer containing 10mM imidazole. The agarose slurry was then transferred to a Biorad polyprep column and the CEA domains eluted from the column by addition of two aliquots of 1 ml of 300mM imidazole in phosphate buffer.

### c. Binding of CEA domain preparations to the scFv immobilised on the sensor chip.

70 *µ*l of each of the four CEA domain preparations (A1-B1, A2-B2, A3-B3 and N) were passed over the scFv-coupled chips at a flow rate of 5 *µ*l/min. After injection of each domain the chip was regenerated by an injection of 10ml of 10mM HCl. For the CEA6 and TO6D11 scFv-coupled chips domains A1-B1, A2-B2 and A3-B3 all resulted in around 100RU's binding to the surfaces. No binding was observed for the N domain on CEA6 or TO6D11 scFv coated chips. K_{off} for the domains were calculated for both CEA6 and TO6D11 scFv coated chips (Table 4) and A3-B3 was found to have the longest off rate, suggesting this domain is the one which is preferentially recognised by CEA6 and TO6D11. Domains A1-B1, A2-B2 and A3-B3 do contain elements which are common to all three domains, which may account for some cross-reactivity of the CEA6 and TO6D11 scFv's with all these domains. This demonstrates that the overall domain recognition characteristics of CEA6 have not been altered in the affinity maturation of this antibody to TO6D11.

As a control a chip with a coupled scFv which has been shown to recognise the N domain of CEA was also tested by passing the different domains across the chip. This scFv gave 54 RU's of binding of the N domain preparation and no detectable binding of the other domains to the scFv, demonstrating the activity of the N domain preparation and reaffirming the specificity of this scFv.

### EXAMPLE 8 - ANALYSIS OF THE BINDING SPECIFICITIES OF CEA1, CEA2, CEA3, CEA4, AND CEA5.

As described in Example 5c CEA1, CEA2, CEA3, CEA4 and CEA5 did not give any detectable ELISA signal when tested for binding to desialylated CEA, suggesting the clones were recognising a sialic acid-containing carbohydrate component of CEA. The specificities of these clones was investigated further as follows.

### a. Testing CEA1, CEA2, CEA3, CEA4 and CEA5 for binding to polysialic acid (PSA) by ELISA.

Biotinylated K1 polysialic acid, a version of PSA which is a polymer of on average approximately 200 monomers of sialic acid, was provided by Dr R Waibel. The K1 version of PSA was purified from the K1 strain of E. coli. E. coli K1 possesses a membranous CMP-NeuAc; poly-α-2-8 sialosyl sialyltransferase complex catalyses the synthesis of long linear PSA (K1) chains.

The PSA was coated onto a streptavidin-coated plate (Pierce, Reacti-Bind) at 10 *µ*g/ml at room temperature for 1 hour. The plate was blocked in 3%MPBS for 1 hour at room temperature and 100*µ*l of monomer preparations of CEA1, CEA2, CEA3, CEA4, CEA5 and CEA6, as a control, in 3%MPBS then added to each well at approximately 100*µ*g/ml. The plate was left at room temperature for 1 hour, then washed three times in PBST, followed by three times in PBS. Detection of bound scFv was with 1 : 200 diluted anti-myc tag antibody (9E10) (Munro and Pelham, 1986) for 1 hour at 37°C. The plate was washed as before and the assay developed with 1 : 5000 diluted alkaline phosphatase conjugated goat anti-mouse IgG (Pierce) at 37°C for 1 hour. Plates were washed as before, rinsed in 0.9% NaCl and the chromagenic substrate pNPP (Sigma) was added. The absorbance was measured at 405nm.

Clones CEA1, CEA2, CEA3, CEA4 and CEA5 all gave a signal on the PSA K1 polymer, whereas CEA6 gave no detectable signal (Figure 13). These results suggest that CEA1, CEA2, CEA3, CEA4 and CEA5 all recognise free K1 PSA and hence that sialic acid plays a role in their recognition of CEA.

### b. Inhibition of binding of CEA1, CEA2, CEA3, CEA4 and CEA5 to CEA by free Kl. or free colonic acid (CA)

Excess free PSA K1 and free PSA CA (at approximately 1 *µ* M) were preincubated with 100*µ*l of the scFv monomer preparations (at approximately 100*µ*g/ml) and the scFv's then used to detect native CEA by ELISA as described in Example 1d(ii). CA is a polymer of sialic acid with an average of approximately 16 sialic acid residues per chain. The signal on native CEA was inhibited to varying extents in the cases of CEA1, CEA2, CEA3, CEA4 and CEA5 for both K1 and CA. Binding of CEA6 to native CEA was not inhibited by the presence of K1 or CA PSA (Figure 14). A summary of the degree of inhibition of scFv binding to CEA by K1 and CA is shown in Table 5. That the binding of CEA1, CEA2, CEA3, CEA4, and to a lower level CEA5, to CEA was inhibited by the free PSA molecules provides further evidence for the recognition of CEA by these scFv an element of sialic acid binding specificity in their recognition for CEA.

### EXAMPLE 9 - IMMUNOCYTOCHEMISTRY OF CEA1, CEA2, CEA3, CEA4, CEA5 AND CEA6 ON NORMAL COLONIC MUCOSA AND COLORECTAL TUMOURS.

Purified monomer preparations of clones CEA1→6 inclusive were used to detect CEA expressed in paraffin-embedded formalin-fixed sections from different tissue sources (BioMedix). Sections were de-waxed in Histoclear, then washed twice with 100% ethanol, once with 70% ethanol, rehydrated in distilled water (all 5 min each) and rinsed in PBST. Endogenous alkaline phosphatase activity was then blocked by incubation with 20% acetic acid for 15 min, rinsed with PBST, then blocked for 1 hour in 1%BSA in PBS (PBSB). After rinsing, monomeric scFv fractions diluted in PBSB were applied and incubated in a humidified atmosphere overnight at 4°C. Slides were rinsed three times with PBST (2 min each), then incubated with 1 : 100 diluted 9E10 in PBSB for 1 hour at room temperature. After rinsing as before, alkaline phosphatase conjugated goat anti-mouse IgG (1 : 100 diluted in PBS/10% foetal calf serum) was added and the incubation continued for 1 hour. Bound antibody was detected with Fast Red (Sigma) substrate, and the section was counterstained with haematoxylin and mounted.

CEA 1, CEA2, CEA3, CEA4 and CEA5 gave weak staining of normal colonic crypt epithelium and heterogeneous staining of the normal surface epithelium. These five clones gave variable positive staining of moderate to well differentiated adenocarcinomas. Staining in the moderately differentiated tumours was localised to the basal surfaces of glands and at the lumenal aspect, while staining in the more well differentiated tumours was confined to the mucin-producing goblet cells. These clones did not give a "classical" anti-CEA staining pattern, which may be explained by their reactivity with carbohydrate elements on CEA which may not be present at all stages of expression.

CEA6 gave intense staining of the normal surface epithelium and goblet cells and crypt epithelium were also reactive. Staining of adenocarcinoma by CEA6 gave uniform intense positivity of moderate to well differentiated tumours, but more heterogeneous staining of poorly differentiated carcinomas.

### EXAMPLE 10 - LOCALISATION OF I¹²⁵-LABELLED ANTI-CEA ANTIBODIES TO HUMAN COLON ADENOCARCINOMA XENOGRAFTS.

Example 6 describes data on the localisation of technetium-99m labelled CEA6 scFv to tumours in a nude mouse xenografted with CEA expressing human colon adenocarcinoma. These experiments have been repeated using I¹²⁵-labelled CEA6 scFv, along with TO6D11 scFv, in the same animal model.

### a. Labelling of monomeric preparations of scFv with I¹²⁵.

Labelling of purified scFv with I¹²⁵ was achieved using the "Iodogen Method" first described by Fraker and Speck (1978) Biochem. Biophysc. Res. Commun 80; 849-857. In this method, iodine is preferentially attached to tyrosine residues in the protein, of which there are four in the VH CDR3 of both CEA6 and TO6D11. Although iodination with CDRs could theoretically compromise antigen binding specificity, both CEA6 and TO6D11 were checked for immunoreactivity by passing them over a CEA affinity column after radiolabelling (column loaned by Dr. David Read, Department of Clinical Oncology, Royal Free Hospital, London). This confirmed that between 7-90% of the labelled protein was able to bind the column, demonstrating that ¹²⁵I labelling is a viable labelling approach for *in vivo* application of the scFv.

### b. Animal model.

Human LS174T xenografts were established in nude mice by subcutaneous passaging from the human colon adenocarcinoma cell line LS174T. Groups of four mice were taken for each time point. The mice were injected through the tail vein with 100*µ*l of approximately 10*µ*g of I¹²⁵-labelled scFv's at a specific activity of 1 mCi/mg. Mice were killed at 3, 24 or 48 hours after injection and the tissue : blood ratios of the antibodies measured by gamma counting.

The results obtained for CEA6 and TO6D11 are shown in Table 6. Both CEA6 and TO6D11 localised to tumour at all time points, again confirming that immunoreactivity had not been compromised. CEA6 gave a tumour : blood of 22.5 : 1 24 hours post-injection and 3.1 : 1 48 hours post-injection. TO6D11 did not localise to the tumour as effectively as CEA6, giving a tumour : blood of 5.8 : 1 24 hours post-injection, but the TO6D11 which did target the tumour was retained there longer than the targeted CEA6; at 48 hours post-injection the tumour : blood for TO6D11 was 6.6 : 1.

### EXAMPLE 11: REFINEMENT OF EPITOPE MAPPING OF ANTIBODIES SPECIFIC FOR CEA

### (a) Selection of specific peptides from a large phage display library

To analyse in detail the sequence(s) on the CEA molecule recognised specifically by CEA6, we used purified monomeric CEA6 scFv as antigen in the selection of a very large (>10¹¹ clones) combinatorial repertoire of peptides displayed on phage. The peptide library used was constructed as described by Fisch et al., 1996, Proc. Natl. Acad. Sci. USA 93: 7761-7766. Briefly, ten random amino acids encoded on a pUC-based plasmid were allowed to recombine *in vivo* with ten amino acids encoded by the acceptor phage and separated by a 5-amino acid spacer. The repertoire of recombined sequences (encoding 25 amino acids) was fused to the gIII protein of filamentous phage and was available for display. Phage were purified from cultures grown overnight at 30°C in 2YT media containing 12.5*µ*g/ml tetracycline. Typical phage yields were in the order of 1 - 5 x 10¹¹ t.u./ml.

CEA6 scFv which had been purified and FPLC fractionated as described in Example 3 was used to coat an Immunotube (Nunc, Maxisorp) at 10*µ* g/ml in PBS, overnight at 4°C. The tube was blocked with PBS containing 3% (w/v) Marvel (PBSM) for lh at room temperature, after which phage-containing supernatants (5 x 10¹¹ t.u.), pre-blocked in PBSM, were allowed to bind for a further 90min at room temp. Non-specific phage were washed away as described in Example 1 and specific phage were eluted with 3ml of 100mM triethylamine, neutralised with 1.5ml 1M Tris Cl pH 8.0 and reinfected into E.coli TG1. Up to four rounds of selection were carried out and the yield of CEA6-specific phage in the polyclonal population was assessed by ELISA as below. Individual phage clones from rounds 3 and 4 of panning were then screened further for specificity by ELISA.

Ninety-six well plates were coated with either CEA6 scFv, or other scFv of different specificity as controls, at 10*µ*g/ml in PBS overnight at 4°C. The plates were blocked with PBSM for lh at room temperature, then supernatant from individual clones which had been grown in microtitre plates in 2YT-tet were pre blocked in PBSM before adding to the CEA6 scFv coated plate. Phage binding was detected by using anti-M13 HRP-conjugate, diluted 1:5000. Detection was with an OPD substrate (Sigma); plates were read at 490nm in a microtitre plate reader.

### (b) Sequencing of clones selected from the peptide library

Sequencing of CEA6-positive phage was performed by PCR of the two exons separately with the following oligonucleotide primers:

Exon.1  Oligo 4445    5'-ACTTGGTTAGGTCCATGTCCGTCAGC-3'
        fdPCRBACK     5'-GCGATGGTTGTTGTCATTGTCGGC-3'

Exon 2  Oligo 3358    5'-GAAGTGATGCAACACTGGAGC-3'

           fdPCRFOR      5'-TAGCCCCCTTATTAGCGTTTGCCA-3'

Table 7 shows a compilation of sequences of peptide phage selected after three and four rounds of panning on CEA6 scFv, shown by ELISA to bind the scFv specifically. Of the clones selected, only 1/11 had recombined as a 25 amino acid peptide; the remainder of clones comprised 10 amino acids encoded by the acceptor phage (exon 2 in Fisch et al., *supra).*

The selected sequences are shown aligned to human CEA sequence common to all three 178 amino acid domain repeats (A1B1, A2B2, A3B3). The residues underlined in Table 7 lie at positions 139-148 inclusive, within the 'A' subdomain, where the strongest homology to selected peptide phage is observed. Peptides P3G12 and P3C8, selected at Pan 3, showed the most striking homology; in addition, clones with the same sequences as P3G12 (P4C5) and P3C8 (P4A3) were also isolated at Pan 4, suggesting positive antigenic selection of phage with these sequences. Phage P3G12 and P3C8 were therefore taken for purification by precipitation with PEG (as described in Example 1(a)) and both further specificity analysis (c) and inhibition of CEA6 binding to CEA (d) by ELISA.

### (C) Specificity analysis using scFv with common frameworks

In order to investigate the specificity of phage P3G12 and P3C8 for CEA6 further, the following purified scFv were taken for coating onto 96 well plates as described in (a) above:
(i) scFv P2-2E10, specific for bovine histone H1, which has the same germline VH (DP10) and VL (L12a) as CEA6 but differs in sequence in the CDRs of both chains;
(ii) scFv P2-1D2, specific for whole bovine histone, with a different VH (DP75, from VH1) but the same VL;
(iii) scFv VoDox-1, specific for the compound doxorubicin, with a different VH (DP47, from VH3) but the same VL.

All scFv were purified as in Example 3 and ELISAs were carried out as described in (a). Figure 15 shows the absorbance plotted for each peptide tested; none of the scFv tested is able to bind specifically to peptides selected on CEA6 scFv. As well as confirming the specificity of both peptides for CEA6, this confirms that neither peptide is recognising either the *myc* tag or the *his* tag present on all scFv. In independent experiments, the selected peptides were also demonstrated to bind CEA6 reformatted as human IgG1, k, but showed no binding to an unrelated human antibody of the same isotype.

### (d) Binding inhibition ELISA using peptide phage

Inhibition of binding of CEA6 scFv to CEA was measured by ELISA using either selected peptide phage, native CEA (positive control) or irrelevant peptide phage (negative control). CEA was coated onto an 96 well plate at 0.5*µ*g/ml, 50*µ*l per well overnight at 37°C. The plate was blocked with 3% MPBS; meanwhile, CEA6 scFv at 1*µ*g/ml was pre-incubated with a tenfold dilution series (in 3% MPBS) of phage from clones P3G12, P3C8 or P4A2, or with CEA (starting at 55nM) for 90min at room temperature. The preincubated samples were then transferred to the blocked plate for lhr at room temperature. Bound CEA6 was detected by standard detection with 9E10 (1*µ*g/ml) followed by anti-mouse IgG alkaline phosphatase conjugate (1/2500) and PNPP substrate (Sigma).

Figure 16 shows the reduction in ELISA signal by different concentrations of both specific and nonspecific phage, compared with inhibition using native CEA. Both P3G12 and P3C8 phage at concentrations >1.0 x 10¹⁰ t.u./ml inhibit CEA binding specifically, whereas an irrelevant phage (P4A2) does not.

### EXAMPLE 12: ANALYSIS OF TUMOUR UPTAKE AND NORMAL TISSUE BIODISTRIBUTION OF ¹²⁵I-LABELLED scFv SPECIFIC FOR CEA

Example 10 describes the efficient localisation of ¹²⁵I-labelled CEA-specific scFv CEA6 and TO6D11 to xenografted human colon tumours. In a separate experiment, it had been shown that all scFv derived from affinity maturation of CEA6 (HBB11, TO6D10, TO6D4, TO6D12, as well as TO6D11 and CEA6) were localised in tumour tissue at 24h post injection. In that experiment, CEA6 and TO6D12 demonstrated the highest tumour:blood ratios measured at 24h (11:1). TO6D4 was lower (4:1), but the percentage of the injected dose in the tumour at 24h was the highest (2%). TO6D4 also gave more favourable tumour:kidney ratios, suggesting slower clearance. Further experiments described were carried out to confirm these observations and give a back-to-back comparison of CEA6, TO6D12 and TO6D4, incorporating a larger number of timepoints at which incorporated radioactivity could be measured.

### a. Labelling of monomeric scFv with ¹²⁵Iodine

Single chain Fv were labelled by the Iodine method, as referenced in Example 10. Following labelling, 90%, 85% and 62% of ¹²⁵I-CEA6, ¹²⁵I-TO6D12 and ¹²⁵I-TO6D4 bound to the CEA-sepharose column, respectively.

### b. Tumour xenograft model

Groups of 16 mice bearing LS174T xenografts were given a single intravenous dose of each iodinated scFv (1*µ*Ci/50*µ*g). Groups of 4 mice were sacrificed at 3, 6, 18 and 24h post injection and tumour plus other tissues removed for gamma counting.

All three scFv localised preferentially to tumour. Peak tumour:blood ratios were 14.6:1 (¹²⁵I-CEA6 at 24h), 6.6:1 (¹²⁵I-TO6D12 at 24h) and 7.4:1 (¹²⁵I-TO6D4 at 18h) . Figure 17 shows the mean percentage of the injected dose per g (%ID/g) of tumour plotted for the various timepoints. At the 24h timepoint, the mean %ID/g was 0.5% (¹²⁵I-CEA6), 0.4% (¹²⁵I-TO6D12) and 1.5% (¹²⁵I-TO6D4). TO6D4 showed the most favourable tumour:kidney ratio, suggesting that this scFv was excreted more slowly. However, measurement of the rates of whole body clearance of the three scFv showed that they were indistinguishable, with an estimated t_{1/2}(*α*) of around 2h. The study therefore demonstrates that although clone TO6D4 does not have the slowest off-rate relative to that of CEA6, it has acquired properties resulting in a lower clearance rate after *in vivo* administration.

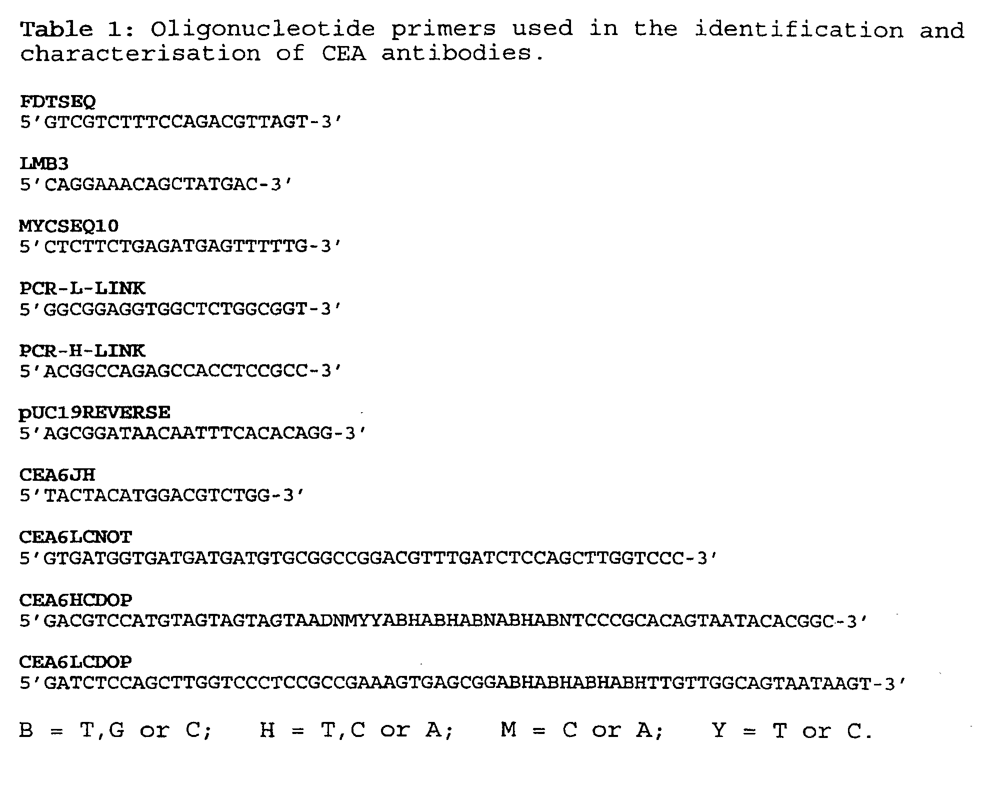

**Table 2:**

| k_{off} and kₒₙ determination of anti-CEA scFvs by surface plasmon resonance. | | | |
|---|---|---|---|
| **Clone** | **k**_{**off**} **(s**^{**-1**}**)** | **k**_{**on**} **(M**^{**-1**}**s**^{**-1**}**)** | **DISSOCIATION CONSTANT (k**_{**off**}**/k**_{**On**}**) (M)** |
| CEA6 | 6.0 X 10⁻³ | 9.0 X 10⁵ | 7.0 X 10⁻⁹ |
| TO6D4 | 4.0 X 10⁻³ | 4.0 X 10⁵ | 1.0 X 10⁻⁸ |
| TO6D8 | 2.3 X 10⁻³ | 4.0 X 10⁵ | 6.0 X 10⁻⁹ |
| TO6D10 | 1.4 X 10⁻³ | 1.0 X 10⁶ | 1.0 X 10⁻⁹ |
| TO6D12 | 3.3 X 10⁻³ | 9.0 X 10⁵ | 3.0 X 10⁻⁹ |
| TO6D11 | 9.0 X 10⁻⁴ | 1.5 X 10⁶ | 6.0 X 10⁻¹⁰ |
| HBB11 | 2.0 X 10⁻³ | 1.5 X 10⁶ | 1.0 X 10⁻⁹ |
| HBA11 | 5.5 X 10⁻³ | 9.0 X 10⁵ | 6.0 X 10⁻⁹ |
| HBB6 | 5.7 X 10⁻³ | 9.0 X 10⁵ | 6.0 X 10⁻⁹ |

**Table 3:**

| Affinities of anti-CEA scFvs measured by binding inhibition ELISA | |
|---|---|
| **Clone** | **DISSOCIATION CONSTANT (M)** |
| CEA1 | 1.0 X 10⁻⁶ |
| CEA2 | 5.0 X 10⁻⁷ |
| CEA3 | 5.0 X 10⁻⁷ |
| CEA4 | 1.0 X 10⁻⁷ |
| CEA5 | 1.0 X 10⁻⁸ |
| CEA6 | 7.0 X 10⁻⁹ |

**Table 4:**

| Relative levels of binding and off rates of purified CEA domains passed across TO6D11 or CEA6 scFv's coupled sensor chip. | | | | | |
|---|---|---|---|---|---|
| **T06D11 375 RU chip** | | | | | |
| | **CEA domains** | | | | |
| | Desial CEA | A1-B1 | A2-B2 | A3-B3 | N |
| RU bound | >1000 | 167 | 112 | 100 | 9 |
| k_{off} (s⁻¹) | 9 x 10⁻³ | 7 x 10⁻² | 7 x 10⁻² | 4 x 10⁻² | - |

| **CEA6 394 RU chip** | | | | | |
|---|---|---|---|---|---|
| | **CEA domains** | | | | |
| | Desial CEA | A1-B1 | A2-B2 | A3-B3 | N |
| RU bound | >1000 | 115 | 130 | 150 | 30 |
| k_{off} (s⁻¹) | 9 x 10⁻³ | 8 x 10⁻² | 8 x 10⁻² | 4 x 10⁻² | - |

Too few RU's of N domain remained on the TO6D11 and CEA6 chips to calculate a k_{off}.

**Table 5:**

| Percent inhibition of the binding of CEA1, CEA2, CEA3, CEA4, CEA5 and CEA6 to CEA by free K1 and CA PSA. | | |
|---|---|---|
| **Clone** | **Inhibition of native** | **CEA binding (%)** |
| | **K1** | **CA** |
| CEA1 | 53 | 94 |
| CEA2 | 90 | 75 |
| CEA3 | 0 | 29 |
| CEA4 | 25 | 48 |
| CEA5 | 2 | 15 |
| CEA6 | 7 | 0 |

**Table 6:**

| Average tissue: blood ratios of CEA6, T06D11 and Colin scFv's in the mouse model of human colon adenocarcinoma. | | | | | | | |
|---|---|---|---|---|---|---|---|
| | | CEA6 | | | TO6D11 | | |
| | | 3 | hr pi 24 | 48 | 3 | hr pi 24 | 48 |
| Blood | | 1 | 1 | 1 | 1 | 1 | 1 |
| Liver | | 0.4 | 0.9 | 0.8 | 0.5 | 0.8 | 0.6 |
| Kidney | | 1.3 | 5.5 | 4.1 | 1.8 | 5.9 | 6.7 |
| Lung | | 0.6 | 1.1 | 0.7 | 0.7 | 1.1 | 0.7 |
| Spleen | | 0.6 | 0.7 | 0.6 | 0.5 | 0.6 | 0.9 |
| Colon | | 0.4 | 0.5 | 0.4 | 0.4 | 0.4 | 0.6 |
| Muscle | | 0.2 | 0.4 | 0.4 | 0.3 | 0.3 | 0.3 |
| Bone | | 0.3 | 0.5 | 0.7 | 0.3 | 0.4 | 0.5 |
| | | | | | | | |
| Tumour | | 0.9 | 22.5 | 3.1 | 1.0 | 5.8 | 6.6 |
| hr pi = Hours post-injection | | | | | | | |

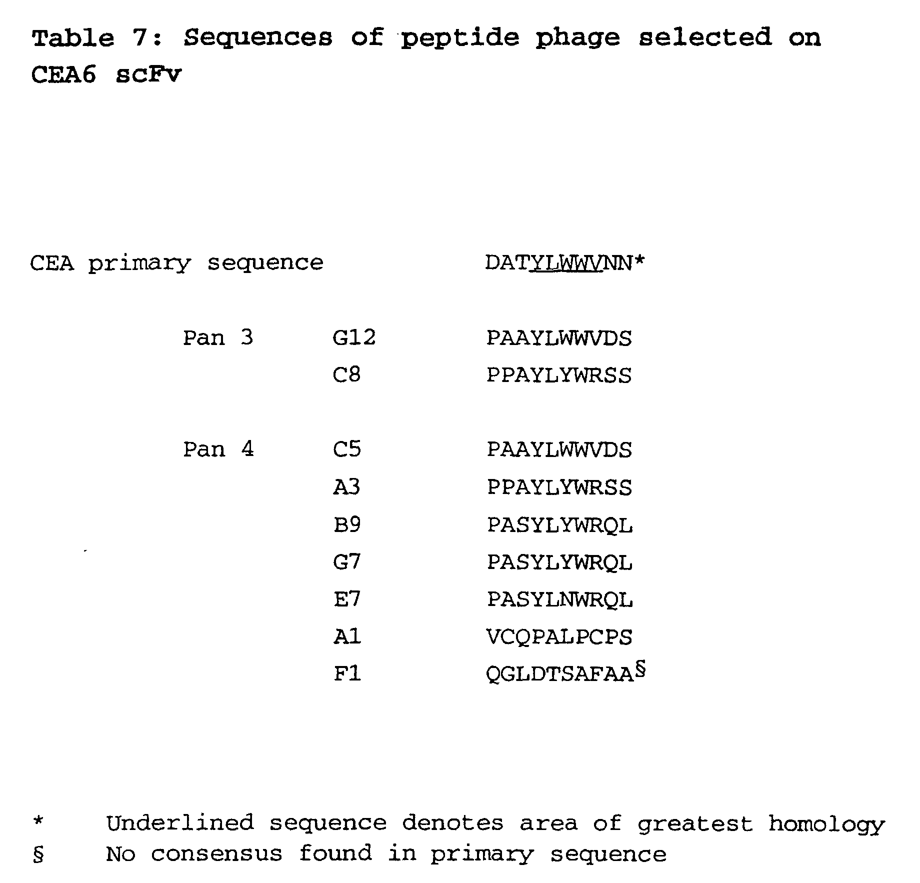

## Claims

1. An isolated specific binding member comprising a human antibody antigen binding domain specific for human carcinoembryonic antigen wherein the binding domain has a dissociation constant for human carcinoembryonic antigen which is less than 1.0 x 10⁻⁸M, is substantially non-cross-reactive with human liver cells, binds to the A3-B3 extracellular domain of human carcinoembryonic antigen and/or binds to cell-associated human carcinoembryonic antigen preferentially over soluble human carcinoembryonic antigen.

2. A specific binding member according to claim 1 wherein the binding domain has a dissociation constant for human carcinoembryonic antigen which is less than 5.0 x 10⁻⁹M.

3. A specific binding member according to claim 1 or claim 2 which does not significantly bind one or more of vascular endothelium, muscle, neutrophils, erythrocytes and lymphocytes.

4. A specific binding member according to any preceding claim wherein the human antibody antigen binding domain comprises a VH domain and a VL domain, the VH and VL domains being selected from the following pairings:
(i) the VH domain of CEA6, the amino acid sequence of which is shown in Figure 1(a), and the VL domain of CEA6, the amino acid sequence for which is shown in Figure 1(b) ;
(ii) the VH domain of CEA6, the amino acid sequence of which is shown in Figure 1(a), and a VL domain selected from T06D4 and T06D12, the amino acid sequences of which are shown in Figure 4;
(iii) that of TO6D11, i.e. the VH domain of TO6D10, the amino acid sequence of which is shown in Figure 2, and the VL domain of TO6D12, the amino acid sequence of which is shown in Figure 4;
(iv) the VH domain of CEA6, the amino acid sequence of which is shown in Figure 1(a), the VL domain of TO6D8, the amino acid sequence of which is shown in Figure 4;
(v) the VL domain of CEA6, the amino acid sequence of which is shown in Figure 1(b), the VH domain of HBB11, the amino acid sequence of which is shown in Figure 2;
(vi) the VL domain of CEA6, the amino acid sequence of which is shown in Figure 1(b), and the VH domain of TO6D10, the amino acid sequence of which is shown in Figure 2;
(vii) the VL domain of CEA6, the amino acid sequence of which is shown in Figure 1(b), and a VH domain selected from HBA11 and HBB6, the amino acid sequences of which are shown in Figure 2;
(viii) the VH domain of CEA6, the amino acid sequence of which is shown in Figure 1(a), and a VL domain selected from LOB1C, LOE17 and LOSC2, the amino acid sequences of which are shown in Figure 3;
(ix) a VH domain which is an amino acid sequence variant of a VH amino acid sequence shown in Figure 1(a) or Figure 2, and a VL domain which has a VL amino acid sequence shown in Figure 1(b), Figure 3 or Figure 4;
(x) a VL domain which is a VL amino acid sequence variant of an amino acid sequence shown in Figure 1(b), Figure 3 or Figure 4, and a VH domain which has a VH amino acid sequence shown in Figure 1(a) or Figure 2; and
(xi) a VH domain which is an amino acid sequence variant of a VH amino acid sequence shown in Figure 1(a) or Figure 2, and a VL domain which is an amino acid sequence variant of a VL amino acid sequence shown in Figure 1(b), Figure 3 or Figure 4.

5. A specific binding member according to claim 1 wherein said human antibody antigen binding domain comprises a VH1, VH3 or VH4 gene sequence of one of the following germ lines: the DP71 germ line; the DP47 germ line; the DP67 germ line; the DP32 germ line; and the DP10 germ line; or a re-arranged form thereof.

6. A specific binding member according to claim 1 wherein said human antibody antigen binding domain comprises a V11, V13 or Vk1 gene sequence of one of the following germ lines: the germ line DPL5; the DPL2 germ line; the germ line DPL16; the germ line L12a; or a re-arranged form thereof.

7. A specific binding member according to claim 1 wherein said human antibody antigen binding domain comprises a VH domain having any of the amino acid sequences shown in Figure 1(a) or is an amino acid sequence variant of any of the VH amino acid sequence shown in Figure 1(a).

8. A specific binding member according to claim 1 wherein said human antibody antigen binding domain comprises one or more complementarity determining region (CDR) with an amino acid sequence identified in Figure 1(a) as a CDR1, CDR2 or CDR3 sequence.

9. A specific binding member according to claim 8 wherein said human antibody antigen binding domain comprises a CDR3 sequence shown in Figure 1(a).

10. A specific binding member according to claim 1 wherein said human antibody antigen binding domain comprises a CDR sequence that is a variant, by way of addition, deletion, substitution or insertion of one or more amino acids, of a CDR sequence identified in Figure 1(a) as a CDR1, CDR2 or CDR3 sequence.

11. A specific binding member according to claim 1 wherein said human antibody antigen binding domain comprises a VH domain which is an amino acid sequence variant of the CEA6 VH.

12. A specific binding member according to claim 1 wherein said human antibody antigen binding domain comprises a VH domain having any of the amino acid sequences shown in Figure 2 as a variant of CEA6 or is an amino acid sequence variant of any of the CEA6 VH variant amino acid sequences shown in Figure 2.

13. A specific binding member according to claim 1 wherein said human antibody antigen binding domain comprises a CDR3 sequence shown in Figure 2 as a variant of CDR3 of CEA6.

14. A specific binding member according to claim 1 wherein said human antibody antigen binding domain comprises a VL domain having any of the amino acid sequences shown in Figure 1(b) or is an amino acid sequence variant of any of the VL amino acid sequences shown in Figure 1(b).

15. A specific binding member according to claim 1 wherein said human antibody antigen binding domain comprises one or more complementarity determining region (CDR) with an amino acid sequence identified in Figure 1(b) as a CDR1, CDR2 or CDR3 sequence.

16. A specific binding member according to claim 1 wherein said human antibody antigen binding domain comprises a CDR sequence that is a variant, by way of addition, deletion, substitution or insertion of one or more amino acids, of a CDR sequence identified in Figure 1(b) as a CDR1, CDR2 or CDR3 sequence.

17. A specific binding member according to claim 1 wherein said human antibody antigen binding domain comprises a VL domain having any of the amino acid sequences shown in Figure 3 as a variant of CEA6 or is an amino acid sequence variant of any of the CEA6 VL variant amino acid sequences shown in Figure 3.

18. A specific binding member according to claim 1 wherein said human antibody antigen binding domain comprises a CDR3 sequence shown in Figure 3 as a variant of CDR3 of CEA6.

19. A specific binding member according to claim 1 wherein said human antibody antigen binding domain comprises a VL domain having any of the amino acid sequences shown in Figure 4 as a variant of CEA6 or is an amino acid sequence variant of a CEA6 VL variant amino acid sequence shown in Figure 4.

20. A specific binding member according to claim 1 wherein said human antibody antigen binding domain comprises one or more complementarity determining region (CDR) with an amino acid sequence identified in Figure 4 as a CDR1, CDR2 or CDR3 sequence variant of a CEA6 CDR.

21. A specific binding member according to claim 4 wherein said human antibody antigen binding domain comprises the VH domain of CEA6, the amino acid sequence of which is shown in Figure 1(a), and the VL domain of CEA6, the amino acid sequence for which is shown in Figure 1(b).

22. A specific binding member according to any preceding claim which is a single chain Fv (scFv) molecule.

23. A specific binding member according to any of the preceding claims which comprises one or more amino acids in addition to those forming said human antibody antigen binding domain.

24. A specific binding member according to any preceding claim which comprises a label or reporter molecule.

25. A specific binding member according to claim 23 wherein the label is radioactive iodine.

26. An in vitro method of determining the presence of a cell expressing human carcinoembryonic antigen, the method comprising contacting cells with a specific binding member according to claim 1 and determining the binding of the specific binding member to the cells.

27. A method comprising causing or allowing binding of a specific binding member according to any of claims 1 to 24 to human carcinoembryonic antigen in vitro.

28. A specific binding member according to any of claims 1 to 24 for use in a method of therapeutic treatment which comprises
administration of the specific binding member to a mammal.

29. Nucleic acid encoding a specific binding member according to any of claims 1 to 23.

30. Nucleic acid according to claim 29 which is part of a vector.

31. A cell containing nucleic acid according to claim 29 or claim 30.

32. A method of production of a specific binding member which includes expression from nucleic acid according to claim 29 or claim 30.

33. A method according to claim 32 which includes culturing a cell according to claim 31.

34. A method according to claim 32 or claim 33 wherein the specific binding member is isolated and/or purified following expression.

35. A method according to claim 32 wherein the specific binding member is used in formulating a composition.

## Patentansprüche

1. Isoliertes spezifisches Bindungselement, umfassend eine Human-Antikörper-Antigen-Bindungsdomäne, die für carcinoembryonales Human-Antigen spezifisch ist, worin die Bindungsdomäne eine Dissoziationskonstante für carcinoembryonales Human-Antigen von unter 1,0 x 10⁻⁸ M aufweist, mit Human-Leberzellen im Wesentlichen nicht kreuzreagiert, sich an die extrazelluläre A3-B3-Domäne von carcinoembryonalem Human-Antigen bindet und/oder sich gegenüber löslichem carcinoembryonalem Human-Antigen bevorzugt an Zell-assoziiertes carcinoembryonales Human-Antigen bindet.

2. Spezifisches Bindungselement nach Anspruch 1, worin die Bindungsdomäne eine Dissoziationskonstante für carcinoembryonales Human-Antigen von unter 5,0 x 10⁻⁹ M aufweist.

3. Spezifisches Bindungselement nach Anspruch 1 oder 2, das sich nicht signifikant an eines oder mehrere von Gefäßendothel, Muskeln, Neutrophile, Erythrozyten und Lymphozyten bindet.

4. Spezifisches Bindungselement nach einem der vorangegangenen Ansprüche, worin die Human-Antikörper-Antigen-Bindungsdomäne eine VH-Domäne und eine VL-Domäne umfasst, wobei die VH- und die VL-Domäne aus den folgenden Paarungen ausgewählt sind:
(i) der VH-Domäne von CEA6, deren Aminosäuresequenz in Fig. 1(a) gezeigt wird, und der VL-Domäne von CEA6, deren Aminosäuresequenz in Fig. 1(b) gezeigt wird;
(ii) der VH-Domäne von CEA6, deren Aminosäuresequenz in Fig. 1(a) gezeigt wird, und einer VL-Domäne, die aus TO6D4 und TO6D12 ausgewählt ist, deren Aminosäuresequenzen in Fig. 4 gezeigt werden;
(iii) jener von TO6D11, d.h. die VH-Domäne von TO6D10, deren Aminosäuresequenz in Fig. 2 gezeigt wird, und der VL-Domäne von TO6D12, deren Aminosäuresequenz in Fig. 4 gezeigt wird;
(iv) der VH-Domäne von CEA6, deren Aminosäuresequenz in Fig. 1(a) gezeigt wird, der VL-Domäne von TO6D8, deren Aminosäuresequenz in Fig. 4 gezeigt wird;
(v) der VL-Domäne von CEA6, deren Aminosäuresequenz in Fig. 1(b) gezeigt wird, der VH-Domäne von HBB11, deren Aminosäuresequenz in Fig. 2 gezeigt wird;
(vi) der VL-Domäne von CEA6, deren Aminosäuresequenz in Fig. 1(b) gezeigt wird, und der VH-Domäne TO6D10, deren Aminosäuresequenz in Fig. 2 gezeigt wird;
(vii) der VL-Domäne von CEA6, deren Aminosäuresequenz in Fig. 1(b) gezeigt wird, und einer VH-Domäne, die aus HBA11 und HBB6 ausgewählt ist, deren Aminosäuresequenzen in Fig. 2 gezeigt werden;
(viii) der VH-Domäne von CEA6, deren Aminosäuresequenz in Fig. 1(a) gezeigt wird, und einer VL-Domäne, die aus LOB1C, LOE17 und LOSC2 ausgewählt ist, deren Aminosäuresequenzen in Fig. 3 gezeigt werden;
(ix) einer VH-Domäne, die eine Aminosäuresequenzvariante einer VH-Aminosäuresequenz ist, die in Fig. 1(a) oder Fig. 2 gezeigt wird, und einer VL-Domäne, die eine VL-Aminosäuresequenz aufweist, die in Fig. 1(b), Fig. 3 oder Fig. 4 gezeigt wird;
(x) einer VL-Domäne, die eine VL-Aminosäuresequenzvariante einer Aminosäuresequenz ist, die in Fig. 1(b), Fig. 3 oder Fig. 4 gezeigt wird, und einer VH-Domäne, die eine VH-Aminosäuresequenz aufweist, die in Fig. 1(a) oder Fig. 2 gezeigt wird; sowie
(xi) einer VH-Domäne, die eine Aminosäuresequenzvariante einer VH-Aminosäuresequenz ist, die Fig. 1(a) oder Fig. 2 gezeigt wird, und einer VL-Domäne, die eine Aminosäuresequenzvariante einer VL-Aminosäuresequenz ist, die in Fig. 1(b), Fig. 3 oder Fig. 4 gezeigt wird.

5. Spezifisches Bindungselement nach Anspruch 1, worin die Human-Antikörper-Antigen-Bindungsdomäne eine VH1-, VH3- oder VH4-Gensequenz einer der folgenden Keimlinien umfasst: der DP71-Keimlinie, der DP47-Keimlinie; der DP67-Keimlinie; der DP32-Keimlinie; und der DP10-Keimlinie; oder einer neugeordneten Form davon.

6. Spezifisches Bindungselement nach Anspruch 1, worin die Human-Antikörper-Antigen-Bindungsdomäne eine VI1-, V13- oder Vk1-Gensequenz einer der folgenden Keimlinien umfasst: der Keimlinie DPL5; der DPL2-Keimlinie, der Keimlinie DPL16; der Keimlinie L12a; oder einer neugeordneten Form davon.

7. Spezifisches Bindungselement nach Anspruch 1, worin die Human-Antikörper-Antigen-Bindungsdomäne eine VH-Domäne umfasst, die eine der in Fig. 1(a) gezeigten Aminosäuresequenzen aufweist, oder eine Aminosäuresequenzvariante einer der in Fig. 1(a) gezeigten VH-Aminosäuresequenzen ist.

8. Spezifisches Bindungselement nach Anspruch 1, worin die Human-Antikörper-Antigen-Bindungsdomäne eine oder mehrere Komplementarität bestimmende Regionen (CDR) mit einer in Fig. 1(a) als CDR1-, CDR-2 oder CDR3-Sequenz identifizierten Aminosäuresequenz umfasst.

9. Spezifisches Bindungselement nach Anspruch 8, worin die Human-Antikörper-Antigen-Bindungsdomäne eine in Fig. 1(a) gezeigte CDR3-Sequenz umfasst.

10. Spezifisches Bindungselement nach Anspruch 1, worin die Human-Antikörper-Antigen-Bindungsdomäne eine CDR-Sequenz umfasst, die eine durch Addition, Deletion, Substitution oder Insertion einer oder mehrerer Aminosäuren erhaltene Variante einer in Fig. 1(a) als CDR1-, CDR2- oder CDR3-Sequenz identifizierten CDR-Sequenz ist.

11. Spezifisches Bindungselement nach Anspruch 1, worin die Human-Antikörper-Antigen-Bindungsdomäne eine VH-Domäne umfasst, die eine Aminosäuresequenzvariante der CEA6 VH ist.

12. Spezifisches Bindungselement nach Anspruch 1, worin die Human-Antikörper-Antigen-Bindungsdomäne eine VH-Domäne umfasst, die eine der in Fig. 2 gezeigten Aminosäuresequenzen als Variante von CEA6 aufweist oder eine Aminosäuresequenzvariante einer der in Fig. 2 gezeigten CEA6 VH-Varianten-Aminosäuresequenzen ist.

13. Spezifisches Bindungselement nach Anspruch 1, worin die Human-Antikörper-Antigen-Bindungsdomäne eine in Fig. 2 als Variante von CDR3 von CEA6 gezeigte CDR3-Sequenz umfasst.

14. Spezifisches Bindungselement nach Anspruch 1, worin die Human-Antikörper-Antigen-Bindungsdomäne eine VL-Domäne umfasst, die eine der in Fig. 1(b) gezeigten Aminosäuresequenzen aufweist oder eine Aminosäuresequenzvariante einer der in Fig. 1(b) gezeigten VL-Aminosäuresequenzen ist.

15. Spezifisches Bindungselement nach Anspruch 1, worin die Human-Antikörper-Antigen-Bindungsdomäne eine oder mehrere Komplementarität bestimmende Regionen (CDR) mit einer in Fig. 1(b) als CDR1-, CDR2- oder CDR3-Sequenz identifizierten Aminosäuresequenz umfasst.

16. Spezifisches Bindungselement nach Anspruch 1, worin die Human-Antikörper-Antigen-Bindungsdomäne eine CDR-Sequenz umfasst, die eine durch Addition, Deletion, Substitution oder Insertion einer oder mehrerer Aminosäuren erhaltene Variante einer in Fig. 1(b) als CDR1-, CDR2- oder CDR3-Sequenz identifizierten CDR-Sequenz ist.

17. Spezifisches Bindungselement nach Anspruch 1, worin die Human-Antikörper-Antigen-Bindungsdomäne eine VL-Domäne umfasst, die eine der in Fig. 3 als Variante von CEA6 gezeigten Aminosäuresequenzen aufweist, oder eine Aminosäuresequenzvariante einer der in Fig. 3 gezeigten CEA6 VL-Varianten-Aminosäuresequenzen ist.

18. Spezifisches Bindungselement nach Anspruch 1, worin die Human-Antikörper-Antigen-Bindungsdomäne eine in Fig. 3 als Variante von CDR2 von CEA6 gezeigte CDR3-Sequenz umfasst.

19. Spezifisches Bindungselement nach Anspruch 1, worin die Human-Antikörper-Antigen-Bindungsdomäne eine VL-Domäne umfasst, die eine der in Fig. 4 als Variante von CEA6 gezeigten Aminosäuresequenzen aufweist, oder eine Aminosäuresequenzvariante einer in Fig. 4 gezeigten CEA6 VL-Varianten-Aminosäuresequenz ist.

20. Spezifisches Bindungselement nach Anspruch 1, worin die Human-Antikörper-Antigen-Bindungsdomäne eine oder mehrere Komplementarität bestimmende Regionen (CDR) mit einer in Fig. 4 als CDR1, CDR2- oder CDR3-Sequenzvariante einer CEA6 CDR identifizierten Aminosäuresequenz umfasst.

21. Spezifisches Bindungselement nach Anspruch 4, worin die Human-Antikörper-Antigen-Bindungsdomäne die VH-Domäne von CEA6, deren Aminosäuresequenz in Fig. 1(a) gezeigt wird, sowie die VL-Domäne von CEA6 umfasst, deren Aminosäuresequenz in Fig. 1(b) gezeigt wird.

22. Spezifisches Bindungselement nach einem der vorangegangenen Ansprüche, das ein Einzelketten-Fv- (scFv-) Molekül ist.

23. Spezifisches Bindungselement nach einem der vorangegangenen Ansprüche, das eine oder mehrere Aminosäuren zusätzlich zu jenen, welche die Human-Antikörper-Antigen-Bindungsdomäne bilden, umfasst.

24. Spezifisches Bindungselement nach einem der vorangegangenen Ansprüche, das ein Marker- oder Reportermolekül umfasst.

25. Spezifisches Bindungselement nach Anspruch 23, worin der Marker radioaktives lod ist.

26. In vitro-Verfahren zum Nachweis der Gegenwart einer Zelle, die carcinoembryonales Human-Antigen exprimiert, wobei das Verfahren das Kontaktieren von Zellen mit einem spezifischen Bindungselement nach Anspruch 1 und das Bestimmen der Bindung des spezifischen Bindungselements an die Zellen umfasst.

27. Verfahren, welches das Veranlassen oder Ermöglichen der Bindung eines spezifischen Bindungselements nach einem der Ansprüche 1 bis 24 an carcinoembryonales Human-Antigen in vitro umfasst.

28. Spezifisches Bindungselement nach einem der Ansprüche 1 bis 24 zur Verwendung in einem Verfahren zur therapeutischen Behandlung, welches die Verabreichung des spezifischen Bindungselements an ein Säugetier umfasst.

29. Nukleinsäure, die für eine spezifisches Bindungselement nach einem der Ansprüche 1 bis 23 kodiert.

30. Nukleinsäure nach Anspruch 29, die Teil eines Vektors ist.

31. Zelle, die Nukleinsäure nach Anspruch 29 oder Anspruch 30 umfasst.

32. Verfahren zur Produktion eines spezifischen Bindungselements, welches die Expression aus Nukleinsäure nach Anspruch 29 oder Anspruch 30 umfasst.

33. Verfahren nach Anspruch 32, welches das Kultivieren einer Zelle nach Anspruch 31 umfasst.

34. Verfahren nach Anspruch 32 oder Anspruch 33, worin das spezifische Bindungselement nach der Expression isoliert und/oder gereinigt wird.

35. Verfahren nach Anspruch 32, worin das spezifische Bindungselement zum Formulieren einer Zusammensetzung verwendet wird.

## Revendications

1. Membre de liaison spécifique isolé comprenant un domaine de liaison d'un antigène d'un anticorps humain spécifique de l'antigène carcinoembryonnaire humain, où le domaine de liaison a une constante de dissociation pour l'antigène carcinoembryonnaire humain qui est inférieure à 1.0 x 10⁻⁸ M, ne réagit sensiblement pas en croisé avec les cellules de foie humain, se lie au domaine extracellulaire A3-B3 de l'antigène carcinoembryonnaire humain et/ou se lie à l'antigène carcinoembryonnaire humain associé aux cellules, préférentiellement par rapport à l'antigène carcinoembryonnaire humain soluble.

2. Membre de liaison spécifique selon la revendication 1 où le domaine de liaison a une constante de dissociation pour l'antigène carcinoembryonnaire humain qui est inférieure à 5,0 x 10⁻⁹ M.

3. Membre de liaison spécifique selon la revendication 1 ou la revendication 2 qui ne se lie pas de manière significative à un ou plusieurs parmi l'endothélium vasculaire, le muscle, les neutrophiles, les érythrocytes et les lymphocytes.

4. Membre de liaison spécifique selon toute revendication précédente où le domaine de liaison de l'antigène de l'anticorps humain comprend un domaine VH et un domaine VL, les domaines VH et VL étant sélectionnés parmi les paires suivantes :
(i) le domaine VH de CEA6, dont la séquence d'acides aminés est montrée à la figure 1(a) et le domaine VL de CEA6, dont la séquence d'acides aminés est montrée à la figure 1(b);
(ii) le domaine VH de CEA6, dont la séquence d'acides aminés est montrée à la figure 1(a) et un domaine VL sélectionné parmi TO6D4 et T06D12, dont les séquences d'acides aminés sont montrées à la figure 4 ;
(iii) celui de TO6D11, à savoir le domaine VH de TO6D10, dont la séquence d'acides aminés est montrée à la figure 2 et le domaine VL de TO6D12, dont la séquence d'acides aminés est montrée à la figure 4 ;
(iv) le domaine VH de CEA6, dont la séquence d'acides aminés est montrée à la figure 1(a), le domaine VL de TO6D8, dont la séquence d'acides aminés est montrée à la figure 4 ;
(v) le domaine VL de CEA6, dont la séquence d'acides aminés est montrée à la figure 1(b), le domaine VH de HBB11, dont la séquence d'acides aminés est montrée à la figure 2 ;
(vi) le domaine VL DE CEA6, dont la séquence d'acides aminés est montrée à la figure 1(b) et le domaine VH de TO6D10, dont la séquence d'acides aminés est montrée à la figure 2 ;
(vii) le domaine VL de CEA6, dont la séquence d'acides aminés est montrée à la figure 1(b) et un domaine VH sélectionné parmi HBA11 et HBB6, dont les séquences d'acides aminés sont montrées à la figure 2 ;
(viii) le domaine VH de CEA6, dont la séquence d'acides aminés est montrée à la figure 1(a) et un domaine VL sélectionné parmi LOB1C, LOE17 et LOSC2, dont les séquences d'acides aminés sont montrées à la figure 3 ;
(ix) un domaine VH qui est une séquence d'acides aminés variante d'une séquence d'acides aminés de VH que l'on peut voir à la figure 1(a) ou à la figure 2 et un domaine VL qui a une séquence d'acides aminés de VL que l'on peut voir à la figure 1(b), la figure 3 ou la figure 4 ;
(x) un domaine VL qui est une variante de la séquence d'acides aminés de VL d'une séquence d'acides aminés montrée à la figure 1(b), la figure 3 ou la figure 4 et un domaine VH qui a une séquence d'acides aminés de VH que l'on peut voir à la figure 1(a) ou à la figure 2 ; et
(xi) un domaine VH qui est une séquence d'acides aminés variante d'une séquence d'acides aminés de VH que l'on peut voir à la figure 1(a) ou à la figure 2, et un domaine VL qui est une séquence d'acides aminés variante d'une séquence d'acides aminés de VL que l'on peut voir à la figure 1(b), la figure 3 ou la figure 4.

5. Membre de liaison spécifique selon la revendication 1 où ledit domaine de liaison d'antigène d'anticorps humain comprend une séquence génétique de VH1, VH3 ou VH4 de l'une des lignées germinales suivantes : la lignée germinale DP71; la lignée germinale DP47; la lignée germinale DP67; la lignée germinale DP32 et la lignée germinale DP10 ou leur forme réarrangée.

6. Membre de liaison spécifique selon la revendication 1 où ledit domaine de liaison d'antigène d'anticorps humain comprend une séquence génétique V11, V13 ou Vk1 de l'une des lignées germinales suivantes : la lignée germinale DPL5; la lignée germinale DPL2; la lignée germinale DPL16; la lignée germinale L12a ou une forme réarrangée de celles-ci.

7. Membre de liaison spécifique selon la revendication 1 où ledit domaine de liaison d'antigène d'anticorps humain comprend un domaine de VH ayant l'une des séquences d'acides aminés montrées à la figure 1(a) ou est une variante de la séquence d'acides aminés de l'une des séquences d'acides aminés de VH que l'on peut voir à la figure 1(a).

8. Membre de liaison spécifique selon la revendication 1 où ledit domaine de liaison d'antigène d'anticorps humain comprend une ou plusieurs régions déterminant la complémentarité (CDR) avec une séquence d'acides aminés identifiée à la figure 1(a) en tant que séquence de CDR1, CDR2 ou CDR3.

9. Membre de liaison spécifique selon la revendication 8 où ledit domaine de liaison d'antigène d'anticorps humain comprend une séquence de CDR3 montrée à la figure 1(a).

10. Membre de liaison spécifique selon la revendication 1 où ledit domaine de liaison d'antigène d'anticorps humain comprend une séquence de CDR3 qui est une variante par addition, délétion, substitution ou insertion d'un ou plusieurs acides aminés d'une séquence de CDR3 identifiée à la figure 1(a) en tant que séquence de CDR1, CDR2 ou CDR3.

11. Membre de liaison spécifique selon la revendication 1 où ledit domaine de liaison d'antigène d'anticorps humain comprend un domaine de VH qui est une séquence d'acides aminés variante de VH de CEA6.

12. Membre de liaison spécifique selon la revendication 1 où ledit domaine de liaison d'antigène d'anticorps humain comprend un domaine de VH ayant l'une des séquences d'acides aminés montrées à la figure 2 en tant que variante de CEA6 ou est une variante de la séquence d'acides aminés de l'une des séquences d'acides aminés variantes de VH de CEA6 que l'on peut voir à la figure 2.

13. Membre de liaison spécifique selon la revendication 1 où ledit domaine de liaison d'antigène d'anticorps humain comprend une séquence de CDR3 montrée à la figure 2 comme variante de CDR3 de CEA6.

14. Membre de liaison spécifique selon la revendication 1 où ledit domaine de liaison d'antigène d'anticorps humain comprend un domaine de VL ayant l'une des séquences d'acides aminés montrées à la figure 1(b) ou est une variante de la séquence d'acides aminés de l'une des séquences d'acides aminés de VL que l'on peut voir à la figure 1(b).

15. Membre de liaison spécifique selon la revendication 1 où ledit domaine de liaison d'antigène d'anticorps humain comprend une ou plusieurs régions déterminant la complémentarité (CDR) avec une séquence d'acides aminés identifiée à la figure 1(b) en tant que séquence de CDR1, CDR2 ou CDR3.

16. Membre de liaison spécifique selon la revendication 1 où ledit domaine de liaison d'antigène d'anticorps humain comprend une séquence de CDR qui est une variante par addition, délétion, substitution ou insertion d'un ou plusieurs acides aminés d'une séquence de CDR identifiée à la figure 1(b) par séquence de CDR1, CDR2 ou CDR3.

17. Membre de liaison spécifique selon la revendication 1 où ledit domaine de liaison d'antigène d'anticorps humain comprend un domaine de VL ayant l'une des séquences d'acides aminés que l'on peut voir à la figure 3 en tant que variante de CEA6 ou est une séquence d'acides aminés variante de l'une des séquences d'acides aminés variantes de VL de CEA6 que l'on peut voir à la figure 3.

18. Membre de liaison spécifique selon la revendication 1 où ledit domaine de liaison d'antigène d'anticorps humain comprend une séquence de CDR3 montrée à la figure 3 en tant que variante de CDR3 de CEA6.

19. Membre de liaison spécifique selon la revendication 1 où ledit domaine de liaison d'antigène d'anticorps humain comprend un domaine de VL ayant l'une des séquences d'acides aminés montrées à la figure 4 en variante de CEA6 ou est une variante de la séquence d'acides aminés d'une séquence d'acides aminés variante de VL de CEA6 de la figure 4.

20. Membre de liaison spécifique selon la revendication 1 où ledit domaine de liaison d'antigène d'anticorps humain comprend une ou plusieurs régions déterminant la complémentarité (CDR) avec une séquence d'acides aminés identifiée à la figure 4 en tant que séquence variante de CDR1, CDR2 ou CDR3 de CDR de CEA6.

21. Membre de liaison spécifique selon la revendication 4 où ledit domaine de liaison d'antigène d'anticorps humain comprend le domaine de VH de CEA6, dont la séquence d'acides aminés est montrée à la figure 1(a) et le domaine de VL de CEA6, dont la séquence d'acides aminés est montrée à la figure 1(b).

22. Membre de liaison spécifique selon toute revendication précédente qui est une molécule de Fv à une seule chaîne (scFv).

23. Membre de liaison spécifique selon l'une quelconque des revendications précédentes qui comprend un ou plusieurs acides aminés, en plus de ceux formant ledit domaine de liaison d'antigène d'anticorps humain.

24. Membre de liaison spécifique selon toute revendication précédente qui comprend un marqueur ou molécule reporteur.

25. Membre de liaison spécifique selon la revendication 23 où le marqueur est de l'iode radioactif.

26. Méthode in vitro pour déterminer la présence d'un antigène carcinoembryonnaire humain exprimant des cellules, la méthode comprenant la mise en contact des cellules avec un membre de liaison spécifique selon la revendication 1 et la détermination de la liaison du membre de liaison spécifique aux cellules.

27. Méthode consistant à forcer ou à permettre la liaison d'un membre de liaison spécifique selon l'une quelconque des revendications 1 à 24 à un antigène carcinoembryonnaire humain in vitro.

28. Membre de liaison spécifique selon l'une quelconque des revendications 1 à 24 à utiliser dans une méthode de traitement thérapeutique qui comprend l'administration du membre de liaison spécifique à un mammifère.

29. Acide nucléique codant pour un membre de liaison spécifique selon l'une quelconque des revendications 1 à 23.

30. Acide nucléique selon la revendication 29 qui fait partie d'un vecteur.

31. Cellule contenant de l'acide nucléique selon la revendication 29 ou la revendication 30.

32. Méthode de production d'un membre de liaison spécifique qui comprend l'expression de l'acide nucléique selon la revendication 29 ou la revendication 30.

33. Méthode selon la revendication 32 qui comprend la culture d'une cellule selon la revendication 31.

34. Méthode selon la revendication 32 ou la revendication 33 où le membre de liaison spécifique est isolé et/ou purifié à la suite de l'expression.

35. Méthode selon la revendication 32 où le membre de liaison spécifique est utilisé pour formuler une composition.
